(19) **Europäisches Patentamt** / **European Patent Office** / **Office européen des brevets**

(11) **EP 4 691 366 A1**

(12) # EUROPEAN PATENT APPLICATION
published in accordance with Art. 153(4) EPC

(43) Date of publication:
**11.02.2026 Bulletin 2026/07**

(21) Application number: **24780582.3**

(22) Date of filing: **27.03.2024**

(51) International Patent Classification (IPC):
**A61B 5/16** (2006.01)   **B25J 13/08** (2006.01)

(52) Cooperative Patent Classification (CPC):
**A61B 5/16; B25J 13/08**

(86) International application number:
**PCT/JP2024/012526**

(87) International publication number:
**WO 2024/204481 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023054113**

(71) Applicant: **Nitto Denko Corporation**
**Ibaraki-shi, Osaka 567-8680 (JP)**

(72) Inventors:
• **FUKUSHIMA, Rihito**
  **Ibaraki-shi, Osaka 567-8680 (JP)**
• **KIYOSHIMA, Keita**
  **Ibaraki-shi, Osaka 567-8680 (JP)**
• **SHIMIZU, Yusuke**
  **Ibaraki-shi, Osaka 567-8680 (JP)**

(74) Representative: **Epping - Hermann - Fischer**
**Patentanwaltsgesellschaft mbH**
**Schloßschmidstraße 5**
**80639 München (DE)**

(54) **PSYCHOLOGICAL STATE INFORMATION ACQUISITION DEVICE AND ROBOT**

(57)   A psychological state information acquisition apparatus is provided for acquiring psychological state information of a user with excellent accuracy. The psychological state information acquisition apparatus includes a camera configured to capture an image of a user, a biological information acquirer configured to acquire biological information of the user by using electromagnetic waves, and an information outputter configured to output psychological state information, which is information related to a psychological state of the user obtained based on the image of the user captured by the camera and the biological information acquired by the biological information acquirer.

FIG.9

EP 4 691 366 A1

**Description**

TECHNICAL FIELD

The present invention relates to a psychological state information acquisition apparatus and a robot.

BACKGROUND ART

For example, Patent Document 1 discloses an emotion identification device having a wireless device and a camera.

RELATED ART DOCUMENTS

PATENT DOCUMENT

Patent Document 1: Japanese Unexamined Patent Application Publication No. 2020-140609

SUMMARY OF THE INVENTION

PROBLEMS TO BE SOLVED BY THE INVENTION

The apparatus described in Patent Document 1 acquires psychological state information, such as emotion information of a user, solely based on radio wave reflections from a wireless device. The accuracy of acquiring information related to user's psychological states can be improved.

The present invention provides a psychological state information acquisition apparatus that demonstrates the accuracy in acquiring user's psychological states, and a robot.

MEANS FOR SOLVING THE PROBLEM

According to one aspect of the present invention, a psychological state information acquisition apparatus includes a camera configured to capture an image of a user, a biological information acquirer configured to acquire biological information of the user by using electromagnetic waves, and an information outputter configured to output psychological state information, which is information related to a psychological state of the user obtained based on the image of the user captured by the camera and the biological information acquired by the biological information acquirer.

EFFECTS OF THE INVENTION

The present invention provides a psychological state information acquisition apparatus capable of demonstrating excellent accuracy in acquiring psychological state information of a user, and a robot.

BRIEF DESCRIPTION OF THE DRAWINGS

[FIG. 1] FIG. 1 is a perspective view of a robot according to an embodiment.

[FIG. 2] FIG. 2 is a side view of the robot according to the embodiment.

[FIG. 3] FIG. 3 is a cross-sectional view taken in line III-III in FIG. 2.

[FIG. 4] FIG. 4 is a diagram illustrating a configuration of a camera according to the embodiment.

[FIG. 5] FIG. 5 is a diagram illustrating a configuration of a vital sensor according to the embodiment.

[FIG. 6] FIG. 6 is a block diagram illustrating a hardware configuration of a controller according to the embodiment.

[FIG. 7] FIG. 7 is a block diagram illustrating a function configuration of a controller according to a first embodiment.

[FIG. 8] FIG. 8 is a block diagram illustrating a hardware configuration of a controller according to the first embodiment.

[FIG. 9] FIG. 9 is a block diagram illustrating a function configuration of a learning device according to the first embodiment.

[FIG. 10] FIG. 10 is a schematic diagram of a trained model of a neuron according to the embodiment.

[FIG. 11] FIG. 11 is a schematic diagram of a trained model of a neural network according to the embodiment.

[FIG. 12] FIG. 12 is a flowchart showing processing in a controller according to a first embodiment.

[FIG. 13] FIG. 13 is a flowchart showing processing in a learning device according to the first embodiment.

[FIG. 14] FIG. 14 is a block diagram illustrating a function configuration of a learning device according to a modified example.

[FIG. 15] FIG. 15 is a flowchart showing processing in the learning device according to the modified example.

[FIG. 16] FIG. 16 is a diagram illustrating an example of a state where a user is away from a robot.

[FIG. 17] FIG. 17 is a diagram illustrating an example of a state where a user's face cannot be seen.

[FIG. 18] FIG. 18 is a diagram illustrating an example of a state where a robot is leaning on a user.

[FIG. 19] FIG. 19 is a block diagram illustrating a function configuration of a controller according to a second embodiment.

[FIG. 20] FIG. 20 is a flowchart showing processing in a controller according to a second embodiment.

DETAILED DESCRIPTION OF THE INVENTION

Embodiments of the present invention will be hereinafter described in detail with reference to the drawings. In each of the drawings, the same reference numerals will be assigned to the same components, and redundant descriptions will be omitted.

The following embodiments exemplify a psychological state information acquisition apparatus and a robot that embodies the technical philosophy of the present invention, and the present invention is not limited to the following embodiments. The dimensions, materials, shapes, relative angles, etc. of the components described below are not intended to limit the scope of the present invention only, but are intended to be exemplary, unless specifically stated. The size, positional relationship, etc. of the components illustrated in the drawings may be exaggerated in order to clarify the description.

A psychological state information acquisition apparatus according to an embodiment includes a camera configured to capture an image of a user; a biological information acquirer configured to acquire biological information of the user by using electromagnetic waves; an information outputter configured to output information related to a psychological state of the user obtained based on the image of the user captured by the camera and the biological information acquired by the biological information acquirer. The psychological state of the user includes emotions such as happiness and sadness, emotion classification based on the Russell ring model, emotion classification based on Plutchik's theory, emotion, stress, tension, fatigue, alertness, sleepiness, relaxation, excitement, etc. The psychological state information acquisition apparatus according to the embodiment can increase the accuracy in acquiring the psychological state information of the user by estimating a psychological state of the user in a multimodal manner by using an image of the user captured by the camera and the biological information of the user. Herein, "multimodal" means acquiring the psychological state information of the user by using multiple types of information.

In the present specification, a robot equipped with a psychological state information acquisition apparatus according to an embodiment will be described as an example. The robot offers therapeutic effects to a user through interactions, for example. The robot determines and performs an action suitable for a psychological state of the user based on psychological state information of the user acquired by the psychological state information acquisition apparatus according to the embodiment, so as to offer therapeutic effects to the user. With enhanced accuracy in acquisition of psychological state information of the user by the psychological state information acquisition apparatus according to the embodiment, the robot can determine an action suitable for a psychological state of the user and deliver enhanced therapeutic effects to the user.

The "user" in this specification means a user of the robot and a user of the psychological state information acquisition apparatus. A typical example of a user is a working person living alone, a senior with grown-up children living on their own, or a frail elderly person who is eligible for home health care. The user may include not only a user of the robot but also a person who needs to make physical contact with the robot, for example a robot coordinator or a robot technician.

The communication between the user and the robot includes non-contact, verbal communications and communications involving touching or tactile interaction. For example, a state in which the user is interacting with the robot while touching the robot by holding the robot without any vocalized words is also considered as a state in which the user and the robot are communicating. The tactile interaction between the robot and the user means an act in which the user and the robot interact in a tactile manner (an act of touching), such as stroking, tapping (touching), embracing, etc. In this specification, the term "hugging" may be replaced with the term "holding".

However, the psychological state information acquisition apparatus according to the embodiment is not limited to the use of the robot, and can be applied to a wide range of applications, such as home electric appliances and vehicles.

<Example of Overall Configuration of Robot 100>

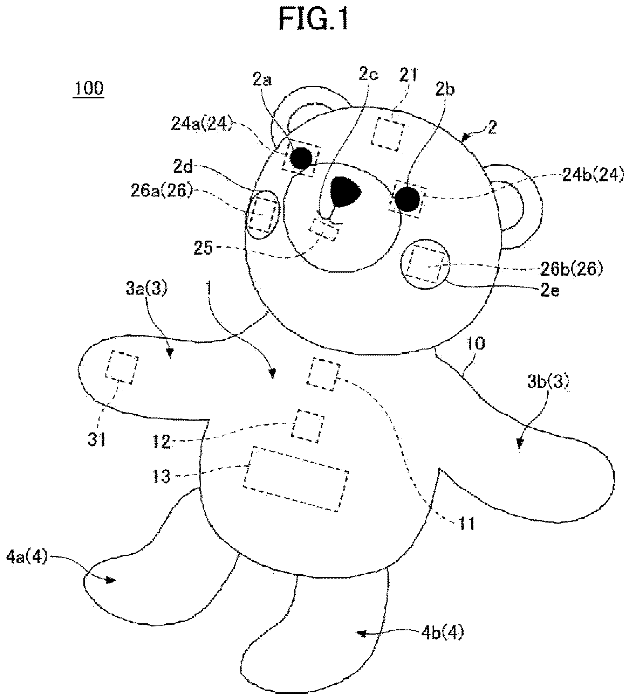

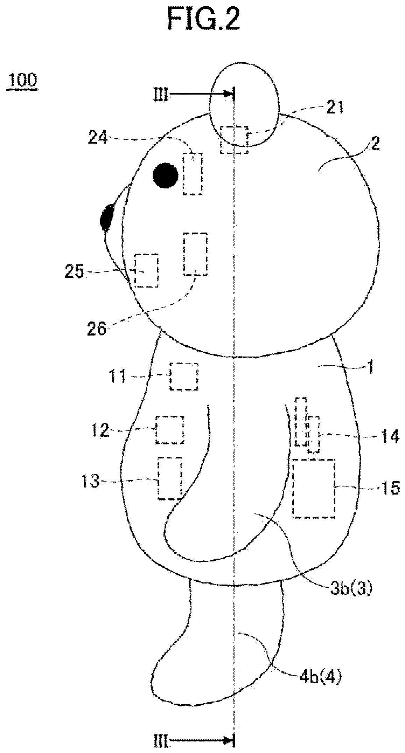

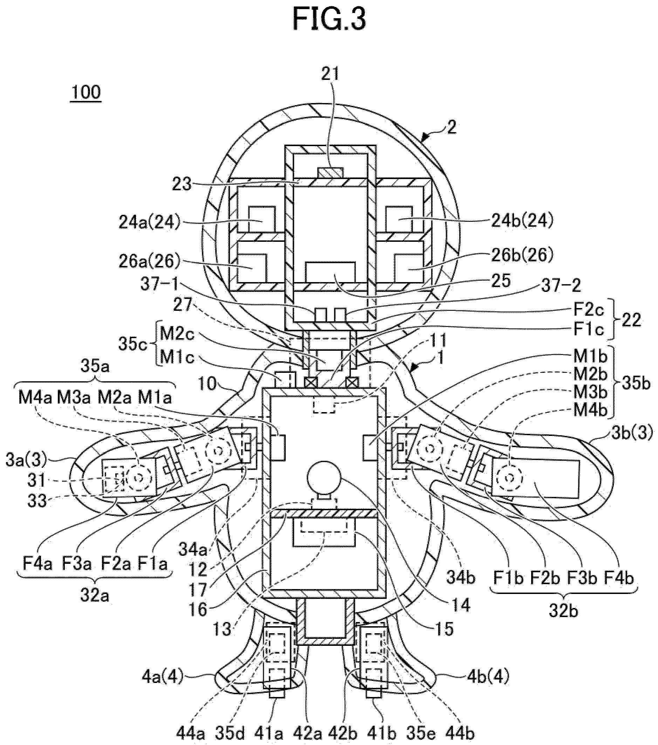

The configuration of a robot 100 according to the embodiment will be described with reference to FIGS. 1 through 3. FIG. 1 is a perspective view illustrating the robot 100 according to the embodiment. FIG. 2 is a side view of the robot 100. FIG. 3 is a cross-sectional view taken in line III-III in FIG. 2.

As illustrated in FIGS. 1 through 3, the robot 100 is, as an example, a teddy bear-shaped robot and capable of communicating with the user. The robot 100 has an exterior member 10 and can be driven by supplied electric power. The robot 100 is made of a size and weight suitable for the user to hold. However, the robot 100 is not limited to a teddy bear-shaped robot and may have various shapes or structures.

The exterior member 10 has softness. The exterior member 10 includes a soft material that is comfortable to touch when the user of the robot 100 touches the robot 100, for example. As the material of the exterior member 10, a material containing an organic material, such as urethane foam, rubber, resin, or fiber, can be used. The exterior member 10 preferably is made of an exterior, such as a urethane foam material having heat insulation properties and a soft cloth material covering the outer surface of the exterior.

The robot 100 has, for example, a torso 1, a head 2, arms 3, and legs 4. The head 2 has a right eye 2a, a left eye 2b, a mouth 2c, a right cheek 2d, and a left cheek 2e. The arms 3 include a right arm 3a and a left arm 3b, and the legs 4 include a right leg 4a and a left leg 4b. Herein, the torso 1 corresponds to a robot body. Each of the head 2, arms 3, and legs 4 corresponds to a driving body which is relatively displaceable to the robot body.

The arms 3 are displaceable with respect to the torso 1. For example, when the robot 100 is held by the user, the right arm 3a and the left arm 3b are displaced and brought into contact with the user's neck, torso, or the like so as to embrace the user. Since this motion evokes a sense of affinity toward the robot 100, it promotes tactile interaction between the user and the robot 100. The tactile interaction between the user and the robot 100 means an act of the user and the robot touching each other, such as stroking, tapping (touching), and hugging.

The torso 1, the head 2, the arms 3, and the legs 4 are all covered by the exterior member 10. The exterior member of the torso 1 and the exterior member of the arms 3 are incorporated, and the exterior member of the head 2 and the exterior member of the legs 4 are separated from the incorporated exterior member of the torso 1 and the arms 3. However, the robot 100 is not limited to these configurations, and for example, only the parts of the robot 100 frequently touched by the user may be covered by the exterior members 10. At least one of the exterior members 10 in each of the torso 1, the head 2, the arms 3, and the legs 4 may be separated from other exterior members. Any of the non-displaceable parts in the head 2, the arms 3, or the legs 4 may be constituted only of the exterior member 10 without including a component, such as a sensor, inside.

The robot 100 has a camera 11, a tactile sensor 12, a controller 13, a vital sensor 14, a battery 15, a first capacitance sensor 21, and a second capacitance sensor 31, all of which are arranged inside the exterior members 10. The camera 11, the tactile sensor 12, the controller 13, the vital sensor 14, and the battery 15 are arranged inside the exterior member 10 in the torso 1. The first capacitance sensor 21 is arranged inside the exterior member 10 in the head 2, and the second capacitance sensor 31 is arranged inside the exterior member 10 in the arm 3.

The robot 100 has a display 24, a speaker 25, and a light 26, all of which are arranged inside the exterior member 10 in the head 2. The robot 100 has the display 24 inside the exterior member 10 in each of the right eye 2a and the left eye 2b. The robot 100 has the speaker 25 inside the exterior member 10 in the mouth 2c and the light 26 inside the exterior member 10 in each of the right cheek 2d and the left cheek 2e. In the present embodiment, the robot 100 further has a first pyroelectric sensor 37-1 and a second pyroelectric sensor 37-2.

More details will be described. As illustrated in FIG. 3, the robot 100 has a torso frame 16 and a torso mounting table 17 inside the exterior member 10 in the torso 1. The robot 100 has a head frame 22 and a head mounting table 23 inside the exterior member 10 in the head 2. Furthermore, the robot 100 has a right arm frame 32a and a right arm mounting table 33 inside the exterior member 10 in the right arm 3a, and has a left arm frame 32b inside the exterior member 10 in the left arm 3b. In addition, the robot 100 has a right leg frame 42a inside the exterior member 10 in the right leg 4a, and has a left leg frame 42b inside the exterior member 10 in the left leg 4b.

The torso frame 16, the head frame 22, the right arm frame 32a, the left arm frame 32b, the right leg frame 42a, and the left leg frame 42b are structures formed by combining a plurality of columnar members. The torso mounting table 17, the head mounting table 23, and the right arm mounting table 33 are plate-like members having mounting surfaces. The torso mounting table 17 is fixed to the torso frame 16, the head mounting table 23 is fixed to the head frame 22, and the right arm mounting table 33 is fixed to the right arm frame 32a. The torso frame 16, the head frame 22, the right arm frame 32a, the left arm frame 32b, the right leg frame 42a, and the left leg frame 42b may be formed in a box shape including a plurality of plate-like members.

The right arm frame 32a is coupled to the torso frame 16 via a right arm coupling mechanism 34a, and can be displaced relative to the torso frame 16 by being driven by a right arm servomotor 35a. When the right arm frame 32a is displaced, the right arm 3a is displaced relative to the torso 1. The right arm coupling mechanism 34a preferably has a reduction gear for increasing the output torque of the right arm servomotor 35a, for example.

In the present embodiment, the right arm frame 32a is composed of an articulated robot arm including a plurality of frame members and a plurality of coupling mechanisms. For example, the right arm frame 32a has a right shoulder frame F4a, a right upper arm frame F3a, a right elbow frame F2a, and a right forearm frame F1a. The torso frame 16, the right shoulder frame F1a, the right upper arm frame F2a, the right elbow frame F3a, and the right forearm frame F4a are coupled to each other via respective coupling mechanisms.

The right arm servomotor 35a is a generic name for a plurality of servomotors. For example, the right arm servomotor 35a includes a right shoulder servomotor M1a, a right upper arm servomotor M2a, a right elbow servomotor M3a, and a right forearm servomotor M4a. The right shoulder servomotor M1a causes the right shoulder frame F1a to rotate around a rotation axis perpendicular to the torso frame 16. The right upper arm servomotor M2a causes the right upper arm frame F2a to rotate around a rotation axis perpendicular to the rotation axis of the right shoulder frame F1a. The right elbow servomotor M3a causes the right elbow frame F3a to rotate around a rotation axis perpendicular to the rotation axis of the right upper arm frame F2a. The right forearm servomotor M4a causes the right forearm frame F4a to rotate around a rotation axis perpendicular to the rotation axis of the right elbow frame F3a.

The left arm frame 32b is coupled to the torso frame 16 via a left arm coupling mechanism 34b, and can be displaced relative to the torso frame 16 by being driven by a left arm servomotor 35b. When the left arm frame 32b is displaced, the left arm 3b is displaced relative to the torso 1. The left arm coupling mechanism 34b preferably has a reduction gear for increasing the output torque of the left arm servomotor 35b, for example.

In the present embodiment, the left arm frame 32b is composed of an articulated robot arm including a plurality of frame members and a plurality of coupling mechanisms. For example, the left arm frame 32b has a left shoulder frame F1b, a left upper arm frame F2b, a left elbow frame F3b, and a left forearm frame F4b. The torso frame 16, the left shoulder frame F1b, the left upper arm frame F2b, the left elbow frame F3b, and the left forearm frame F4b are coupled to each other via respective coupling mechanisms.

The left arm servomotor 35b is a generic name for a plurality of servomotors. For example, the left arm servomotor 35b includes a left shoulder servomotor M1b, a left upper arm servomotor M2b, a left elbow servomotor M3b, and a left forearm servomotor M4b. The left shoulder servomotor M1b causes the left shoulder frame F1b to rotate around a rotation axis perpendicular to the torso frame 16. The left upper arm servomotor M2b causes the left upper arm frame F2b to rotate around a rotation axis perpendicular to the rotation axis of the left shoulder frame F1b. The left elbow servomotor M3b causes the left elbow frame F3b to rotate around a rotation axis perpendicular to the rotation axis of the left upper arm frame F2b. The left forearm servomotor M4b causes the left upper arm frame F4b to rotate around a rotation axis perpendicular to the rotation axis of the left elbow frame F3b.

With the arms 3 having a four-axis joint in the above-described manner, the robot 100 can realize a more realistic motion. For example, the robot 100 can perform an action of reassuring the user by moving the arm 3 to "tap" or "stroke" the user who has feelings of fear or anxiety. The robot 100 can convey empathy with the user by moving the arm 3 to "hug" or "raise both arms for" the user who is experiencing positive emotions.

The head frame 22 is coupled to the torso frame 16 via a head coupling mechanism 27, and can be displaced relative to the torso frame 16 by being driven by a head servomotor 35c. When the head frame 22 is displaced, the head 2 is displaced relative to the torso 1. The head coupling mechanism 27 preferably has a reduction gear for increasing the output torque of the head servomotor 35c, for example.

The head frame 22 has a neck frame F1c and a face frame F2c. The torso frame 16, the neck frame F1c, and the face frame F2c are coupled to each other via a coupling mechanism.

The head servomotor 35c is a generic name for a plurality of servomotors. For example, the head servomotor 35c has a neck servomotor M1c and a face servomotor M2c. The neck servomotor M1c causes the neck frame F1c to rotate around a rotation axis perpendicular to the torso frame 16. The face servomotor M2c causes the head frame F2c to rotate around a rotation axis perpendicular to the rotation axis of the neck frame F1c.

With the head 2 having a two-axis joint in the above-described manner, the robot 100 can realize a motion with higher reality. For example, the robot 100 moves the head 2 to "look up" (pay attention) to the user who has an angry feeling so as to exhibit an action of being concerned about the user.

The right leg frame 42a is coupled to the torso frame 16 via the right leg coupling mechanism 44a, and has a right leg wheel 41a on the bottom surface. In order to stabilize the posture of the robot 100, the robot 100 preferably has two right leg wheels 41a in the front-rear direction of the right leg frame 42a. The right leg wheel 41a is driven by the right leg servomotor 35d and can rotate around a rotation axis perpendicular to the front-rear direction of the right leg frame 42a. The rotation of the right leg wheel 41a enables the robot 100 to travel. The right leg coupling mechanism 44a preferably has a reduction gear for increasing the output torque of the right leg servomotor 35d, for example.

The left leg frame 42b is coupled to the torso frame 16 via a left leg coupling mechanism 44b, and has a left leg wheel 41b on the bottom surface. In order to stabilize the posture of the robot 100, the robot 100 preferably has two left leg wheels 41b in the front-rear direction of the left leg frame 42b. The left leg wheel 41b is driven by a left leg servomotor 35e and can rotate around a rotation axis perpendicular to the front-rear direction of the left leg frame 42b. The rotation of the left leg wheel 41b enables the robot 100 to travel. The left leg coupling mechanism 44b preferably has a reduction gear for increasing the output torque of the left leg servomotor 35e, for example.

In the present embodiment, the robot 100 moves forward or backward by simultaneously rotating the right leg wheel 41a and the left leg wheel 41b forward or backward. By applying a brake on one of the right leg wheel 41a or the left leg wheel 41b and moving the other forward or backward, the robot 100 turns right or left.

With the legs 4, the robot 100 can realize a motion with higher reality. For example, the robot 100 can convey empathy with the user by moving the legs 4 and the arms 3 to hug the user who is experiencing positive emotions.

The camera 11 is fixed to the torso frame 16. The tactile sensor 12, the controller 13, the vital sensor 14, and the battery 15 are fixed to the torso mounting table 17. The controller 13 and the battery 15 are fixed to the side opposite to the side where the tactile sensor 12 and the vital sensor 14 are fixed to the torso mounting table 17. The arrangement of the controller 13 and the battery 15 depends on the space in the torso mounting table 17 and is not necessarily limited to the above-described example. In the case where the battery 15 is fixed to the side opposite to the side where the tactile sensor 12 and the vital sensor 14 are fixed to the torso mounting table 17, the center of gravity of the robot 100 is lowered because the battery 15 is heavier than the other components. A lower center of gravity of the robot 100 is preferable because it is easy to stabilize at least either the position or the posture of the robot 100 and it is easy to at least either charge or replace the battery 15.

The first capacitance sensor 21 is fixed to the head mounting table 23, and the second capacitance sensor 31 is fixed to the right arm mounting table 33. The display 24 has a right eye display 24a and a left eye display 24b. The right eye display 24a, the left eye display 24b, and the speaker 25 are fixed to the head frame 22. The light 26 has a right cheek light 26a and a left cheek light 26b. The right cheek light 26a and the left cheek light 26b are fixed to the head frame 22. The first pyroelectric sensor 37-1 and the second pyroelectric sensor 37-2 are fixed to the head mounting table 23.

The camera 11, the tactile sensor 12, the controller 13, the vital sensor 14, the battery 15, the first capacitance sensor 21, the second capacitance sensor 31, and the like can be fixed by a screw member or an adhesive member. The right eye display 24a, the left eye display 24b, the speaker 25, the right cheek light 26a, the left cheek light 26b, and the like can be fixed by screw members or adhesive members.

There are no particular restrictions on the materials of the torso frame 16, the torso mounting table 17, the head frame 22, the head mounting table 23, the right arm frame 32a, the right arm mounting table 33, and the left arm frame 32b, and resin materials or metal materials can be used. From the viewpoint of securing strength during driving the robot, however, it is preferable to use metal materials, such as aluminum, for the torso frame 16, the right arm frame 32a, and the left arm frame 32b. On the other hand, as long as strength can be secured, it is preferable to use resin materials for the materials of these parts in order to reduce the weight of the robot 100. There are no particular restrictions on the materials of the torso mounting table 17, the head frame 22, the head mounting table 23, the right arm mounting table 33, and the left arm frame 32b, and resin materials or metal materials can be used. From the viewpoint of reducing the weight of the robot 100, it is preferable to use resin materials.

The controller 13 is connected to the camera 11, the tactile sensor 12, the vital sensor 14, the first capacitance sensor 21, the second capacitance sensor 31, the right arm servomotor 35a, and the left arm servomotor 35b so that they can communicate with each other via wired or wireless means. The controller 13 is also connected to the head servomotor 35c, the right leg servomotor 35d, and the left leg servomotor 35e so that they can communicate with each other via wired or wireless means. The controller 13 is also connected to the right eye display 24a, the left eye display 24b, the speaker 25, the right cheek light 26a, and the left cheek light 26b so that they can communicate with each other via wired or wireless means.

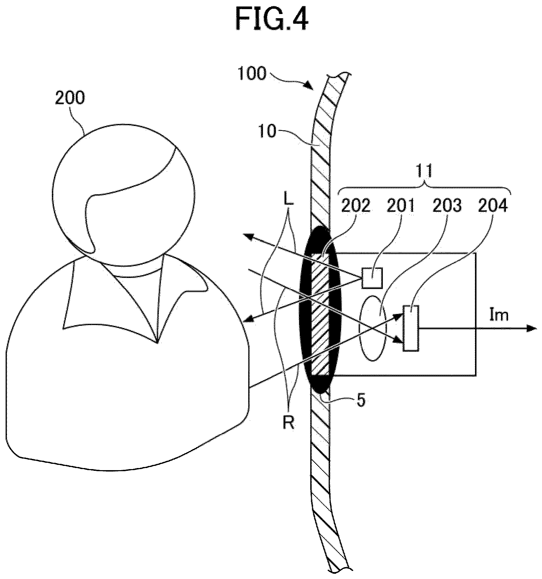

The camera 11 is an image sensor that outputs an image of the robot 100 and its vicinity to the controller 13. The camera 11 can capture an image of the user. The camera 11 is arranged at a position corresponding to the nose 5 of the teddy bear inside the exterior member 10. The camera 11 can be fixed by an adhesive member or the like. The configuration of the camera 11 will be described later in detail with reference to FIG. 4.

The tactile sensor 12 is a sensor element that detects information sensed by the tactile sense of a human hand, converts the information into a tactile signal which is an electric signal, and outputs the signal to the controller 13. For example, the tactile sensor 12 converts information of pressure and vibration generated by a user's touching the robot 100 into a tactile signal using a piezoelectric element, and outputs the signal to the controller 13. The tactile signal that is output from the tactile sensor 12 is used to detect the user 200 touching the robot 100 or presence of the user 200 near the robot 100.

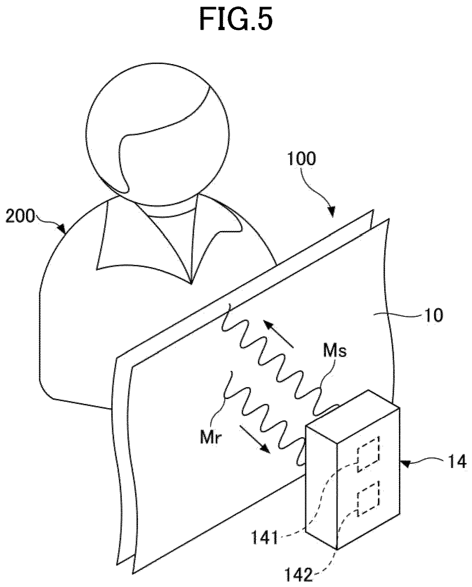

The vital sensor 14 is an example of a biological information acquirer that acquires biological information of a user using electromagnetic waves. The biological information of a user includes information, such as heart rate, respiration rate, pulse wave, and blood pressure. The vital sensor 14 will be described later in detail with reference to FIG. 5.

The first capacitance sensor 21 and the second capacitance sensor 31 are sensor elements that output a capacitance signal to the controller 13, which is detected based on a change in capacitance caused by user's touching the robot 100 or user's proximity to the robot 100. The first capacitance sensor 21 is preferably a rigid sensor without flexibility from the viewpoint of stabilizing the exterior member 10. Since the arms 3 are an easy part for the user to touch, the second capacitance sensor 31 is preferably a flexible sensor, such as a conductive thread or the like from the viewpoint of improving tactile comfort. The capacitance signals that are output from the first capacitance sensor 21 and the second capacitance sensor 31 are used to detect the user's presence or proximity to the robot 100.

Each of the first pyroelectric sensor 37-1 and the second pyroelectric sensor 37-2 is a sensor element for detecting a change in heat (infrared ray) emitted from a living body such as a human body. In the present embodiment, each of the first pyroelectric sensor 37-1 and the second pyroelectric sensor 37-2 is used to acquire information related to distance to the user. For example, the first pyroelectric sensor 37-1 outputs a first pyroelectric signal, which is a detection signal of the user present within a region separated from the robot 100 by a distance of a threshold value or greater, to the controller 13 as distance information. The second pyroelectric sensor 37-2 outputs a second pyroelectric signal, which is a detection signal of the user present within a region separated from the robot 100 by a distance less than a threshold value, to the controller 13 as distance information. When the first pyroelectric signal is obtained from the first pyroelectric sensor 37-1, the controller 13 can recognize that the user is present within a region separated by a distance of a threshold value or greater from the robot 100. When the second pyroelectric signal is obtained from the second pyroelectric sensor 37-2, the controller 13 can recognize that the user is present within a region separated by a distance less than a threshold value from the robot 100. Thus, the robot 100 can obtain a distance to the user based on the first pyroelectric signal from the first pyroelectric sensor 37-1 and the second pyroelectric signal from the second pyroelectric sensor 37-2. In the present embodiment, a distance to the user can be measured with a simple setup of the first pyroelectric sensor 37-1 and the second pyroelectric sensor 37-2, without the need for complex processing.

The right eye display 24a and the left eye display 24b are display modules which display character strings, such as letters, numbers, and symbols, or images in response to commands from the controller 13. The right eye display 24a and the left eye display 24b are configured by, for example, a liquid crystal display module, respectively. The character strings or images displayed on the right eye display 24a and the left eye display 24b are used to express a psychological state of the robot 100. For example, the robot 100 can convey empathy with the user who is experiencing positive emotions by displaying an image of a smiling face on the right eye display 24a and the left eye display 24b and changing its facial expression (making the same facial expression as the user). The robot 100 can convey empathy with the user who is experiencing sad emotions by displaying an image of a crying face on the right eye display 24a and the left eye display 24b and changing its facial expression (making the same facial expression as the user).

The speaker 25 is a speaker unit that amplifies audio signals from the controller 13 and outputs audio sound. The audio sound that is output from the speaker 25 is spoken words or calls of the robot 100 and is used to convey emotional states, simulating psychological expression. For example, the robot 100 can perform an action of cheering up the user who has a sad feeling by speaking out loud through the speaker 25 (encouraging the user by saying "cheer up").

The right cheek light 26a and the left cheek light 26b are light modules that blink or change color in response to on/off signals from the controller 13. The right cheek light 26a and the left cheek light 26b are composed of, for example, LED (light emitting diode) light modules. Blinking or changing color of the right cheek light 26a and the left cheek light 26b is used to convey as-if emotional states, simulating psychological expression. For example, when the user is experiencing positive emotions, the robot 100 can perform an action with empathy with the user by making the right cheek light 26a and the left cheek light 26b blink in yellow.

The battery 15 is a power supply that supplies power to the camera 11, the tactile sensor 12, the controller 13, the vital sensor 14, the first capacitance sensor 21, the second capacitance sensor 31, the right arm servomotor 35a, and the left arm servomotor 35b, respectively. The battery 15 also supplies power to the head servomotor 35c, the right leg servomotor 35d, and the left leg servomotor 35e. The battery 15 also supplies power to the right eye display 24a, the left eye display 24b, the speaker 25, the right cheek light 26a, and the left cheek light 26b. Various secondary batteries, such as lithium-ion batteries and lithium-polymer batteries, can be used for the battery 15.

The various sensors, such as the tactile sensor 12, the first capacitance sensor 21, and the second capacitance sensor 31 in the robot 100, are not essential components. It suffices that the robot 100 has at least the camera 11 and the vital sensor 14. The mounting positions of these sensors can be freely changed. Furthermore, the various sensors, such as the camera 11 and the vital sensor 14, may be arranged outside the robot 100 and transmit necessary information to the robot 100 or an external device wirelessly. For example, a learning device composed of a personal computer (PC) or a server is an example of an external device.

The robot 100 does not need to have the controller 13 inside the exterior member 10, and the controller 13 may communicate with each device wirelessly from outside the exterior member 10. The battery 15 may also supply power to each component from outside the exterior member 10.

In the present embodiment, the head 2, the arms 3, and the legs 4 are displaceable, but the present embodiment is not limited to the example; at least one of the head 2, the arms 3, and the legs 4 may be displaceable. The arms 3 are each composed of a 4-axis articulated robot arm, or may be composed of a 6-axis articulated robot arm. Preferably, the arms 3 are configured to allow coupling with an end effector such as a hand. The legs 4 are composed of a wheel system, but they can be composed of a crawler system or a leg system.

The configuration and shape of the robot 100 are not limited to those exemplified in the present embodiment, and can be appropriately changed according to the user's preference and the mode of use of the robot 100. For example, the robot 100 may be in the form of a robot arm such as an industrial robot, or in the form of a humanoid, rather than in the form of a teddy bear. The robot 100 may be in the form of a mobile device, such as a drone or a vehicle having at least one of arms, a display, a speaker, or a light.

<Configuration Example of Camera 11>

FIG. 4 illustrates an example of the configuration of the camera 11. The camera 11 includes a photographing light source 201, a near-infrared light path filter 202, a lens 203, and an imaging device 204. The camera 11 is arranged near the surface of the robot 100 while being camouflaged by the near-infrared light path filter 202 so as not to be conspicuous from the outside of the robot 100.

The photographing light source 201 irradiates the user 200 with irradiation light L having a wavelength of 360 nm to 2500 nm. The irradiation light L having a wavelength of 360 nm to 2500 nm is light from the visible range to the near-infrared range. In particular, light having a wavelength of 360 nm to 760 nm is called "visible light", and light having a wavelength of 760 nm to 2500 nm is called "near-infrared light". The camera 11 can capture an image of the user 200 using light in a wide wavelength range by capturing the user 200 using the irradiation light L. As the light source of the wavelength, for example, white LEDs, other monochromatic LEDs, near-infrared LEDs, and the like can be used.

The near-infrared light path filter 202 shields visible light and transmits near-infrared light. The near-infrared light path filter 202 is an optical element that transmits light in the near-infrared wavelength range in the irradiation light L from the photographing light source 201. In the present specification, the term "shading" means that the linear transmittance of at least visible light is 20% or less. In the evaluation of shading, the linear transmittance is preferably 10% or less, more preferably 1% or less. The term "transmission" means that the linear transmittance is at least 50% or more. In the evaluation of transmission, the linear transmittance is preferably 80% or less, more preferably 90% or less. The linear transmittance is the ratio of unscattered light obtained from a difference value obtained by subtracting a diffuse transmittance from a total transmittance of irradiation light L having a wavelength of 360 nm to 2500 nm.

The lens 203 forms an image of the user 200 or the like on the imaging surface of the imaging device 204 using reflected light R from the user 200 or the like of the irradiation light L from the photographing light source 201. The imaging device 204 outputs an image Im acquired by capturing the image formed by the lens 203 to the controller 13. A CCD (charge coupled device) or CMOS (complementary metal-oxide semiconductor) or the like can be used as the imaging device. The CCD or CMOS may be a near-infrared camera whose measurement sensitivity is adjusted for imaging in the near-infrared wavelength range. The captured image may be either a still image or a moving image. In the case where the captured image is a near-infrared image, the psychological state of the user may be estimated using the near-infrared image.

The camera 11 can capture an image of the user 200 by sensing the light reflected from the user 200 and transmitted through the near-infrared light path filter 202 within the illumination from the photographing light source 201. The nose 5 is a part that the user 200 less frequently touches than the torso 1, the head 2, and the arms 3. Therefore, by arranging the camera 11 on the nose 5, the user 200 is less likely to touch the camera 11. Thus, when the user 200 touches the robot 100, it is possible to reduce an uncomfortable feeling caused by the surface of the camera 11 being harder than the surface of the exterior member 10. Even if the user 200 touches the nose 5, an uncomfortable feeling of the nose 5 is not so unnatural even if the tactile feeling of the nose 5 is different from that of the torso 1, the head 2, the arms 3, etc.; therefore, the uncomfortable feeling of the touch can be reduced.

The position where the camera 11 is arranged is not limited to the nose 5, but may be other parts such as the mouth and eyes, if the same effect as that of the arrangement on the nose 5 is obtained. The camera 11 is not limited to be arranged inside the exterior member 10, but may be arranged outside the exterior member 10. In the case where the camera 11 is arranged outside the exterior member 10, it is not always necessary to camouflage the camera 11 so that it is less visually recognizable, and therefore the camera 11 need not have the near-infrared light path filter 202 for camouflage.

An image Im captured by the camera 11 may be used for personal authentication of the user 200. A plurality of cameras having the same purpose or different purposes may be respectively provided at a plurality of parts of the robot 100.

In the present embodiment, an image Im captured by the camera 11 is used to estimate the psychological state of the user. For example, the captured image Im includes a face image of the user. The robot 100 estimates the psychological state of the user from the facial expression of the user. Alternatively, the robot 100 uses the face image of the user and estimates a psychological state of the user by remote photoplethysmography (rPPG) based on the captured image Im.

In the present embodiment, an image Im captured by the camera 11 may be used as selection information related to at least one of presence of a user's motion, a distance to the user, detection of the user's face, or a relative angle with respect to the user. Herein, the selection information is information used for multi-modal estimation of a psychological state of the user. For example, in order to estimate a psychological state of the user, the selection information is used to select whether an image of the user captured by the camera or biological information of the user is to be used for estimating a psychological state of the user, or to assign weights to a captured image and biological information to emphasize one over the other. The camera 11 corresponds to an example of a selection information acquirer for acquiring the selection information.

The selection information is not limited to an image Im captured by the camera 11, and any information may be used as long as the above purpose can be fulfilled. For example, biological information of the user acquired by the vital sensor 14 and information related to a distance to the user obtained by using the first pyroelectric signal from the first pyroelectric sensor 37-1 and the second pyroelectric signal from the second pyroelectric sensor 37-2 may be used as the selection information. The selection information acquirer is not limited to the camera 11, and may be the vital sensor 14, the first pyroelectric sensor 37-1, the second pyroelectric sensor 37-2, or the like.

<Configuration Example of Vital Sensor 14>

FIG. 5 is a diagram illustrating the configuration of the vital sensor 14. The vital sensor 14 is a microwave Doppler sensor having a microwave emitter 141 and a microwave receiver 142. Microwaves are an example of electromagnetic waves.

The vital sensor 14 emits a microwave emission wave Ms toward the user 200 from the inside of the exterior member 10 of the robot 100 by the microwave emitter 141. The vital sensor 14 also receives a reflected wave Mr from which the emission wave Ms is reflected by the user 200 by the microwave receiver 142.

Using the Doppler effect, the vital sensor 14 detects, from a difference between the frequency of the emission wave Ms and the frequency of the reflected wave Mr, a minute displacement that occurs on the body surface of the user 200 due to the heartbeat the like in a non-contact manner. The vital sensor 14 can acquire biological information of the user 200, such as heart rate, respiration rate, pulse wave, blood pressure and the like from the detected minute displacement, and can output the acquired biological information to the controller 13. The biological information includes information related to at least one of heartbeat, respiration, blood pressure, or body temperature of the user. For example, the biological information includes at least one of the heart rate [bpm], respiration rate [breaths per minute], blood pressure value [mmHg], or body temperature [°C] of the user.

However, the vital sensor 14 is not limited to a microwave Doppler sensor, and may detect a minute displacement occurring on the body surface by using a change in the coupling between the human body and an antenna, or may use electromagnetic waves other than microwaves such as near-infrared light. The vital sensor 14 may be a millimeter-wave radar, a microwave radar, a CW (continuous wave) radar, a FMCW (frequency modulated continuous wave) radar, a spread spectrum radar or the like. In addition to the Doppler sensor, the vital sensor 14 preferably includes a non-contact thermometer for detecting infrared rays or the like emitted from the user 200. In this case, the vital sensor 14 detects biological information of the user 200 including information related to at least one of heart rate (pulse), respiration, blood pressure, or body temperature.

In the present embodiment, since the vital sensor 14 is provided inside the exterior member 10, the user 200 cannot visually see the vital sensor 14. This suppresses user resistance to biological information detection, facilitating smooth acquisition of biological information. Since the vital sensor 14 can acquire biological information in a non-contact manner, the vital sensor 14 can acquire the biological information even if the user moves to some extent, unlike a contact-type sensor in which the user is required to touch the same place for a certain period of time.

In the present embodiment, the biological information acquired by the vital sensor 14 is used to estimate a psychological state of the user. In the present embodiment, the biological information acquired by the vital sensor 14 may be used as selection information related to at least one of heart rate (pulse), respiration, blood pressure, or body temperature. The vital sensor 14 corresponds to an example of a selection information acquirer for acquiring the selection information.

In the present embodiment, the vital sensor 14 may acquire biological information using electromagnetic waves having a frequency of 300 MHz or more and 300 GHz or less. By using electromagnetic waves having a frequency of 300 MHz or more and 300 GHz or less, it is possible to increase the accuracy in detecting a minute displacement occurring on the body surface of the user 200, to detect biological information signals having a long period, and to increase the resolution in detection of a minute displacement occurring on the body surface of the user 200.

In the present embodiment, promotion of tactile interaction between the user 200 and the robot 100 through an embracing motion of the robot 100 allows the vital sensor 14 to acquire biological information while being in contact with or close to the user 200, with the robot 100 being held by the user 200. Thus, the vital sensor 14 can acquire biological information with high reliability and reduced noise.

<Configuration Example of Controller 13>

(Hardware Configuration Example of the Controller 13)

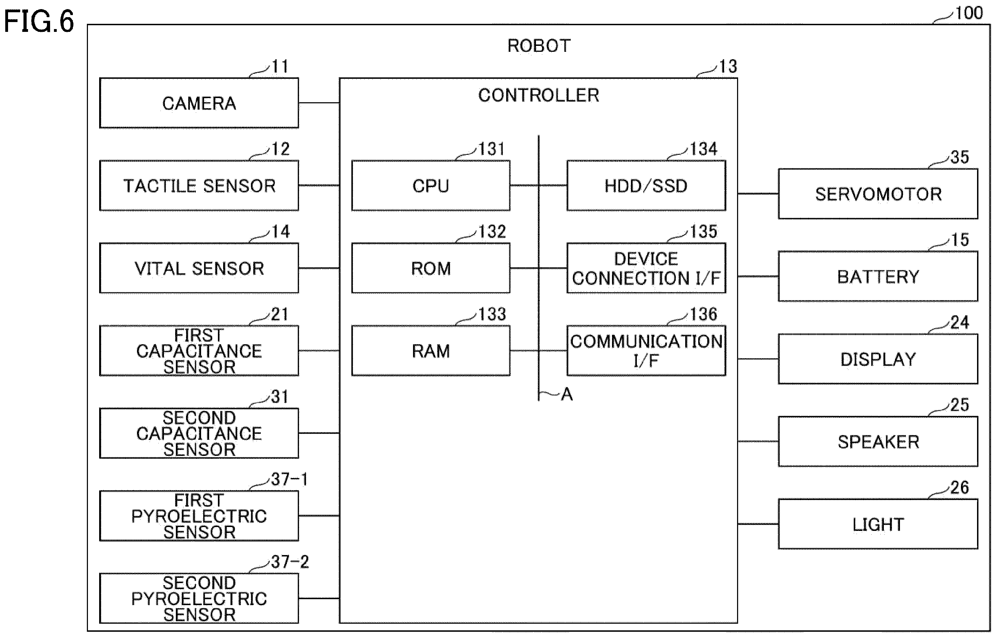

FIG. 6 is a block diagram illustrating an example of the hardware configuration of the controller 13. The controller 13 is constructed by a computer and includes a CPU (central processing unit) 131, ROM (read only memory) 132, and RAM (random access memory) 133. The controller 13 also includes an HDD/SSD (hard disk drive/solid state drive) 134, a device connection I/F (interface) 135, and a communication I/F 136. These are connected to each other through a system bus A so that they can communicate with each other.

The CPU 131 executes control processing including various arithmetic processing. The ROM 132 stores programs used to drive the CPU 131, such as IPL (initial program loader). The RAM 133 is used as a work area of the CPU 131. The HDD/SSD 134 stores various information, such as programs, images captured by the camera 11, and detection information by various sensors, such as biological information acquired by the vital sensor 14.

The device connection I/F 135 is an interface for connecting the controller 13 to various external devices. The external devices include the camera 11, a tactile sensor 12, the vital sensor 14, the first capacitance sensor 21, the second capacitance sensor 31, the servomotor 35, and the battery 15. The external devices also include the display 24, the speaker 25, and the light 26.

Herein, the servomotor 35 is a generic name for the right arm servomotor 35a, the left arm servomotor 35b, the head servomotor 35c, the right leg servomotor 35d, and the left leg servomotor 35e. The display 24 is a generic name for the right eye display 24a and the left eye display 24b. The light 26 is a generic name for the right cheek light 26a and the left cheek light 26b.

The communication I/F 136 is an interface for communicating with an external device via a communication network or the like. For example, the controller 13 connects to the Internet via the communication I/F 136 and communicates with an external device via the Internet.

At least a part of the function implemented by the CPU 131 may be implemented by an electric circuit or an electronic circuit.

[First Embodiment]

(Example of Functional Configuration of Controller 13)

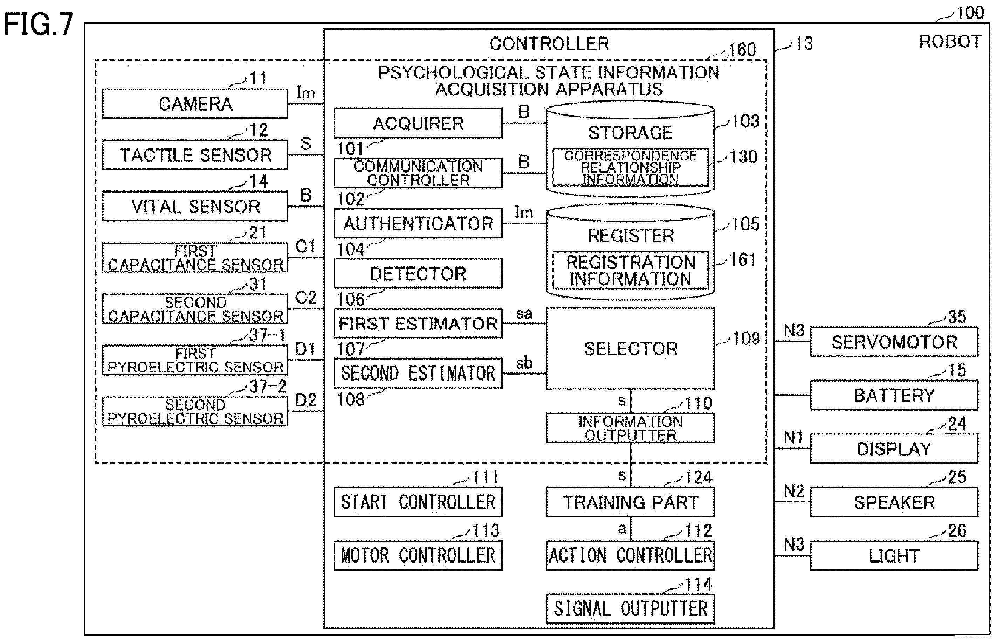

FIG. 7 is a block diagram illustrating an example of a function configuration of the controller 13 according to the first embodiment. The controller 13 includes an acquirer 101, a communication controller 102, a storage 103, an authenticator 104, a register 105, and a detector 106. The controller 13 also includes a first estimator 107, a second estimator 108, a selector 109, an information outputter 110, a training part 124, a start controller 111, an action controller 112, a motor controller 113, and a signal outputter 114.

In the present embodiment, at least a part of the robot 100 constitutes a psychological state information acquisition apparatus 160. For example, the psychological state information acquisition apparatus 160 includes the camera 11, the tactile sensor 12, the vital sensor 14, the first capacitance sensor 21, the second capacitance sensor 31, the first pyroelectric sensor 37-1, and the second pyroelectric sensor 37-2. The psychological state information acquisition apparatus 160 includes the acquirer 101, the communication controller 102, the storage 103, the authenticator 104, the register 105, and the detector 106. Furthermore, the psychological state information acquisition apparatus 160 includes the first estimator 107, the second estimator 108, the selector 109, and the information outputter 110. The psychological state information acquisition apparatus 160 may further include components other than those described above included in the robot 100. The psychological state information acquisition apparatus 160 does not need to include the tactile sensor 12, the first capacitance sensor 21, the second capacitance sensor 31, the first pyroelectric sensor 37-1, and the second pyroelectric sensor 37-2.

The functions of the acquirer 101, the information outputter 110, and the signal outputter 114 can be realized by the device connection I/F 135 or the like. The function of the communication controller 102 can be realized by the communication I/F 136 or the like. The functions of the storage 103 and the register 105 can be realized by nonvolatile memory such as the HDD/SSD 134 or the like. Furthermore, the functions of the authenticator 104, the start controller 111, and the motor controller 113 can be realized by a processor, such as the CPU 131 executing a process specified by a program stored in nonvolatile memory, such as the ROM 132 or the like.

Each of the functions of the detector 106, the first estimator 107, the second estimator 108, the selector 109, the training part 124, and the action controller 112 can be realized by a processor, such as the CPU 131 executing a process specified in a program stored in nonvolatile memory, such as the ROM 132. A part of the function of the controller 13 may be realized by an external device such as a PC or a server, or may be realized by distributed processing between the controller 13 and the external device. For example, the first estimator 107, the second estimator 108, the selector 109, the information outputter 110, and the training part 124 may be configured as a learning device communicably connected to the robot 100.

The acquirer 101 acquires a captured image Im of the user 200 from the camera 11 by controlling communication between the controller 13 and the camera 11. The acquirer 101 acquires a tactile signal S from the tactile sensor 12 by controlling communication between the controller 13 and the tactile sensor 12. Furthermore, the acquirer 101 acquires biological information B of the user 200 from the vital sensor 14 by controlling communication between the controller 13 and the vital sensor 14.

The acquirer 101 acquires a first capacitance signal C1 from the first capacitance sensor 21 by controlling communication between the controller 13 and the first capacitance sensor 21. The acquirer 101 acquires a second capacitance signal C2 from the second capacitance sensor 31 by controlling communication between the controller 13 and the second capacitance sensor 31.

The acquirer 101 acquires a first pyroelectric signal D1 from the first pyroelectric sensor 37-1 by controlling communication between the controller 13 and the first pyroelectric sensor 37-1. The acquirer 101 acquires a second pyroelectric signal D2 from the second pyroelectric sensor 37-2 by controlling communication between the controller 13 and the second pyroelectric sensor 37-2.

The communication controller 102 controls communication with an external device via a communication network or the like. For example, the communication controller 102 can transmit an image Im captured by the camera 11, the biological information B acquired by the vital sensor 14, and the tactile signal S acquired by the tactile sensor 12 to an external device (for example, a learning device, which is described later) via a communication network.

The storage 103 stores the biological information B acquired by the vital sensor 14. The storage 103 continuously stores the acquired biological information B while the acquirer 101 acquires the biological information B from the vital sensor 14. The storage 103 can also store information obtained from an image Im captured by the camera 11, the tactile signal S from the tactile sensor 12, the first capacitance signal C1 from the first capacitance sensor 21, the second capacitance signal C2 from the second capacitance sensor 31, the first pyroelectric signal D1 from the first pyroelectric sensor 37-1, and the second pyroelectric signal C2 from the second pyroelectric sensor 37-2.

The authenticator 104 performs personal authentication of the user. For example, the authenticator 104 performs face authentication by referring to the registration information 161 of the face image registered in advance in the register 105 based on the captured image Im including the face of the user 200 captured by the camera 11. The controller 13 and the psychological state information acquisition apparatus 160 can thereby associate the user 200 currently in contact with or near the robot 100 with the previously registered personal information, and associate the biological information B acquired by the vital sensor 14 with the personal information. The controller 13 can also control to stop the acquisition of the biological information by the vital sensor 14 when the face image included in the captured image Im is not registered in the register 105.

The authentication method by the authenticator 104 is not limited to face authentication. For example, personal authentication may be performed using information related to fingerprints, veins, iris, or voiceprints acquired by the camera 11, the tactile sensor 12, the vital sensor 14, the first capacitance sensor 21, the second capacitance sensor 31, the first pyroelectric sensor 37-1, or the second pyroelectric sensor 37-2. The robot 100 may have a configuration for reading and writing data to and from a tag or a card using wireless communication or electromagnetic induction, and the authenticator 104 may perform personal authentication using information acquired by this configuration.

The detector 106 detects the presence or proximity of the user 200 around the robot 100 based on the image Im captured by the camera 11, etc. The detector 106 preferably detects a distance from the robot 100 to the user 200 based on the image Im captured by the camera 11 (distance image). The detector 106 may also detect the proximity or presence of the user 200 to the robot 100 based on the first capacitance signal C1 or the second capacitance signal C2. Furthermore, the detector 106 detects the user 200 touching the robot 100 or presence of the user 200 within the proximity of the robot 100 based on the tactile signal S from the tactile sensor 12.

The first estimator 107 estimates a psychological state of the user 200 from the image Im captured by the camera 11 and outputs first psychological state information sa. In the present embodiment, the first estimator 107 estimates the first psychological state information sa by a first trained model to which the image Im captured by the camera 11 is input and which outputs the first psychological state information sa. For example, the first estimator 107 estimates a psychological state of the user 200 based on the captured image Im (face image) of the user 200. In the present embodiment, the first estimator 107 performs reinforcement learning to estimate psychological state information st (t is time) of the user 200. However, the first estimator 107 may perform other machine learning, such as supervised learning, semi-supervised learning, or unsupervised learning, to estimate the psychological state information st of the user 200.

The second estimator 108 estimates a psychological state of the user from the biological information B and outputs second psychological state information sb. In the present embodiment, the second estimator 108 estimates the second psychological state information sb by a second trained model to which the biological information B acquired by the vital sensor 14 is input and which outputs the second psychological state information sb. For example, the second estimator 108 estimates a psychological state of the user 200 based on the biological information B acquired by the vital sensor 14. In the present embodiment, the second estimator 108 performs reinforcement learning to estimate psychological state information st (t is time) of the user 200. However, the second estimator 108 may perform other machine learning, such as supervised learning, semi-supervised learning, or unsupervised learning, to estimate the psychological state information st of the user 200.

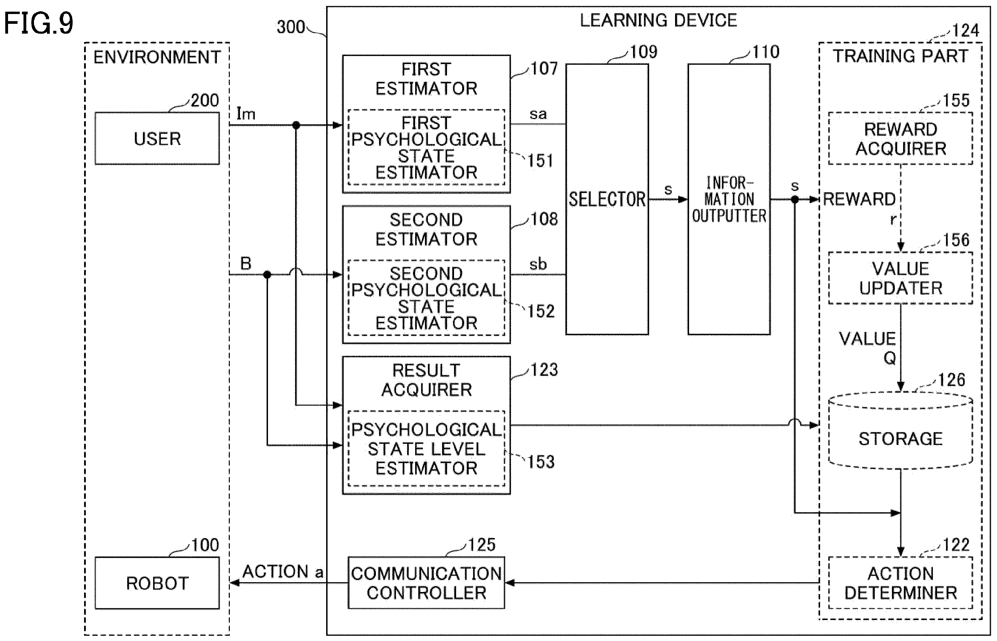

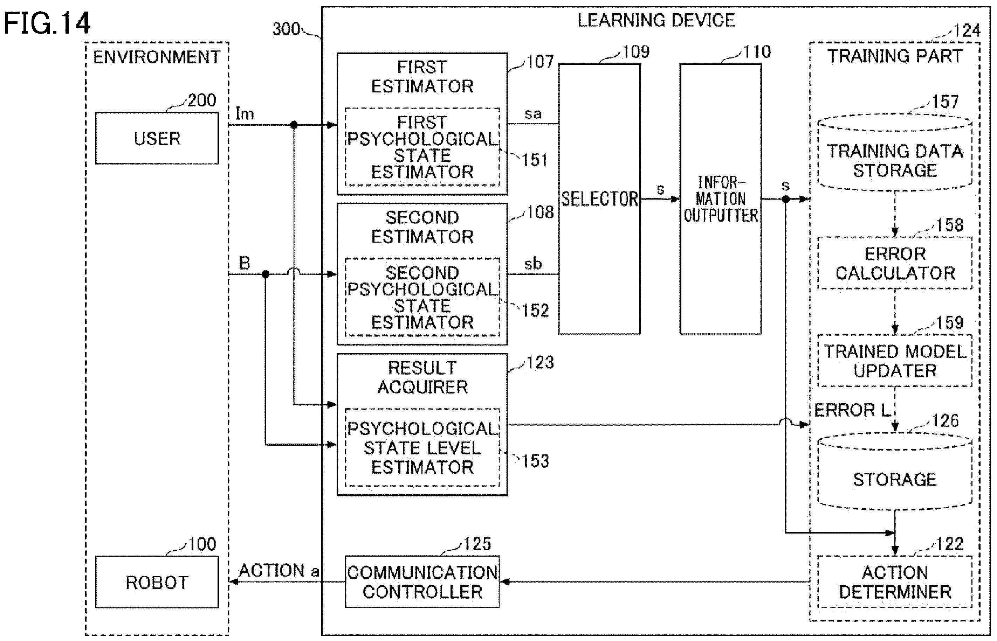

The configuration of the learning device that performs reinforcement learning will be described later in detail with reference to FIG. 9. The configuration of the learning device that performs supervised learning will be described later in detail with reference to FIG. 14. Furthermore, when the training has converged, the learning device may estimate the psychological state information st of the user 200 using a trained model (in the present embodiment, a trained action value table or a trained neural network).

Based on the selection information, the selector 109 considers the first psychological state information sa and the second psychological state information sb to determine the psychological state information s of the user 200. Table 1 shown below is a table explaining the criteria for selecting between the first psychological state information sa and the second psychological state information sb for each decision factor considered by the selector 109.

[Table 1]

| Decision Factors | 1st Psychological State Information based on Captured Image | 2nd Psychological State Information based on Biological Information |
|---|---|---|
| Presence/ Absence of User's Motion | ○ (Motion can be captured as moving image) | × (Body motion affects waveform) |
| Distance to user | ○ (Accuracy would not be reduced regardless of distance. Accuracy is decreased if the number of pixels is too small) | △ (Long distance appears as noise and accuracy is decreased) |
| Presence/ Absence of Detected Face | × (Face cannot be detected if user is looking backward or face is too close) | O (Appropriate as far as body is captured. Data may vary depending on locations) |
| Relative Angle with respect to User | △ (Accuracy changes in accordance with relative angle) | O (Not dependent on relative angle) |

As shown in Table 1, the decision factors include the presence or absence of the user's motion, a distance to the user, the presence or absence of detection of the user's face, and a relative angle with respect to the user. The selector 109 can obtain a decision factor by performing, for example, image processing on the captured image Im as the selection information.

In Table 1, "presence or absence of detection of user's face" corresponds to "detected" if, for example, the captured image Im includes the face of the user 200 at a size larger than a predetermined size threshold. On the other hand, if the captured image Im includes the face of the user 200 with a size smaller than a predetermined size threshold, "presence or absence of detection of user's face" corresponds to "not detected". The "relative angle with respect to the user" includes a relative angle between the user 200 and the robot 100 or the psychological state information acquisition apparatus 160 in the plane orthogonal to the direction of distance from the user.

The symbols "o", "×", and "Δ" indicated in each column of the first psychological state information sa and the second psychological state information sb indicate the suitability evaluation for use in estimation. "○" means appropriate, "×" means inappropriate, and "Δ" means between appropriate and inappropriate. The parentheses shown below "o", "×" and "Δ" indicate the main reason for selecting "○", "×" or "Δ" as the suitability evaluation.

The selector 109 can select either the first psychological state information sa or the second psychological state information sb as the psychological state information s of the user 200 by referring to Table 1, based on the decision factor obtained based on the selection information, such as the captured image Im. Alternatively, the selector 109 may assign weights to the captured image and the biological information to emphasize one over the other by referring to Table 1, based on the decision factor obtained based on the selection information, such as the captured image Im. The selector 109 may select either the weighted first psychological state information sa or the weighted second psychological state information sb as the psychological state information s of the user 200. Furthermore, the selector 109 may consider the first psychological state information sa and the second psychological state information sb to select the psychological state information s of the user 200.

The information outputter 110 outputs the psychological state information s of the user 200 obtained based on the image Im of the user 200 captured by the camera 11 and the biological information B acquired by the vital sensor 14. In the present embodiment, the information outputter 110 outputs the psychological state information s to the information outputter 110. However, the information outputter 110 may output the psychological state information s to an external device by controlling communication between the controller 13 and the external device.

The training part 124 will be described later in detail with reference to FIG. 9.

The start controller 111 causes the vital sensor 14 to start acquiring the biological information B. For example, in the case where the user 200 touching or proximity of the user 200 to the robot 100 is detected by the detector 106, the start controller 111 turns on a switch for supplying power from the battery 15 to the vital sensor 14. The start controller 111 thereby causes the vital sensor 14 to start acquiring the biological information B.

The action controller 112 commands the motor controller 113 or the signal outputter 114 to execute an action at of the robot 100. The action controller 112 also executes a predetermined action at that elicits a favorable response of the user 200 in accordance with the psychological state information st of the user 200. Actions that may cause discomfort can be reduced by commanding the robot 100 to perform an action at that aligns with a psychological state of the user 200. Consequently, the user's engagement with the robot 100 can be sustained, allowing the robot 100 to deliver therapeutic benefits.

The predetermined action at of the robot 100 is preferably an action that reassures the user 200, cheers up, or shows affection, for example, "hug", "tap", or "stroke". The predetermined action at of the robot 100 is preferably an action that pays attention (worries or cares) to the user 200, for example, "look up at the face" or "move the eyes to follow the user". Furthermore, the predetermined action at of the robot 100 is preferably an action of empathizing with the user 200, such as "giving a high-five", "giving backchannel responses", "making the same facial expression", or "making sad voice". The predetermined action at of the robot 100 is preferably an action of playing antics with or playing about with the user 200 such as "giving a paw" or "wiggling out".

The storage 103 stores predefined information related to the action an of the robot 100. The information related to the action an of the robot 100 is managed, for example, by a table of a database. Tables 2A and 2B are examples of an action table TB1 related to the action an of the robot 100. The action table TB1 contains the action IDs identifying actions an of the robot 100, action content of the robot 100, command content of action an, action time per one cycle, and example of use.

[Table 2A]

| Action ID | Action Content | Command Content | Action Time | Example of Use |
|---|---|---|---|---|
| a0 | Hug (Hold to embrace user with arms) | 35: Tracking command + Teaching command Rotation speed 100 mm/s | 10 s/cycle | Cheer up Reassure Show affection |
| a1 | Tap (Touch user's body (side of body, leg, or arm) with arm) | 35: Tracking command + Teaching command Rotation speed 100 mm/s | 5 s/cycle | Cheer up Reassure |
| a2 | Stroke (Stroke user's body (side of body, leg, or arm) with arm) | 35: Tracking command + Teaching command Rotation speed 100 mm/s | 3 s/cycle | Cheer up Reassure |
| a3 | Pat (Gently hit user's body (side of body, leg, or arm) with a vertical stroke) | 35: Tracking command + Teaching command Rotation speed 200 mm/s | 3 s/cycle | Cheer up |
| a4 | Pat (Gently hit user's body (side of body, leg, arm) with a horizontal stroke) | 35: Tracking command + Teaching command Rotation speed 200 mm/s | 3 s/cycle | Cheer up |

(continued)

| Action ID | Action Content | Command Content | Action Time | Example of Use |
|---|---|---|---|---|
| a5 | Push (Push user away) | 35: Tracking command + Teaching command Rotation speed 100 mm/s | 2 s/cycle | Play/play antics |
| a6 | Hold the hand (Hold user's hand with robot hand) | 35: Tracking command + Teaching command Rotation speed 100 mm/s | 5 s/cycle | Reassure |
| a7 | Look up at the face | 35: Tracking command + Teaching command Rotation speed 100 mm/s | 10 s/cycle | Pay attention (Worry or care) |
| a8 | Rub with face (Rub face or nose against user's body) | 35: Tracking command + Teaching command Rotation speed 100 mm/s | 5 s/cycle | Show affection |
| a9 | Lean on (Lean on user's body on the back) | 35: Tracking command + Teaching command Rotation speed 100 mm/s | 10 s/cycle | Show affection |
| a10 | Stroke (Stroke user's head) | 35: Tracking command + Teaching command Rotation speed 100 mm/s | 3 s/cycle | Cheer up |
| a11 | Wiggle out (Fidget when in the same posture for a long time, wiggle out from a cuddle) | 35: Tracking command + Teaching command Rotation speed 200 mm/s | 5 s/cycle | Play/play antics |
| a12 | Give a paw (Put a paw on user's hand) | 35: Tracking command + Teaching command Rotation speed 100 mm/s | 3 s/cycle | Play/play antics |
| a13 | Give a high-five (Raise hand to slap palms) | 35: Tracking command + Teaching command Rotation speed 200 mm/s | 3 s/cycle | Play/play antics Empathize |
| a14 | Give backchannel responses (Nod one's head) | 35: Rotation angle ± 10 degrees Rotation speed 100 mm/s | 2 s/cycle | Empathize |
| a15 | Spread arms (Spread arms so that user can hug easily) | 35: Teaching command Rotation speed 100 mm/s | 5 s/cycle | Show affection Play/play antics |

[Table 2B]

| Action ID | Action Content | Command Content | Action Time | Example of Use |
|---|---|---|---|---|
| a16 | Be scared (Shake, put hands on the head to protect) | 35: Teaching command Rotation speed 200 mm/s | 3 s/cycle | Show reaction (Reaction in response to user's emotion (anger)) |
| a17 | Flail one's hands (Swing one's hands up and down with open palms) | 35: Teaching command Rotation speed 200 mm/s | 3 s/cycle | Play/play antics |
| a18 | Make sound (Make calling voice, worrying voice, sad voice, or confused voice) | 25: Audio output | 2 s/cycle | Explicitly empathize |
| a19 | Change facial expression 1 (Blink or change color of cheeks) | 26: Blinking/ color change | 5 s/cycle | Implicitly empathize |

(continued)

| Action ID | Action Content | Command Content | Action Time | Example of Use |
|---|---|---|---|---|
| a20 | Change facial expression 2 (Make same expression, e.g., smile with face, cry) | 24: Image display | 3 s/cycle | Explicitly empathize |
| a21 | Change facial expression 3 (Open and close eyes) | 24: Image display (Or command to move eye lids) | 2 s/cycle | Show reaction (Reaction to user's emotion (surprise or anger)) |
| a22 | Change facial expression 4 (Change size of pupils or irises, make eyes bloodshot) | 24: Image display | 2 s/cycle | Show reaction (Reaction to user's emotion (surprise or anger)) |
| a23 | Change facial expression 5 (Display or move nose, mouth, eyebrows, or ears) | 24: Image display | 3 s/cycle | Show reaction (Reaction to user's emotion) |
| a24 | Change facial expression 6 (Move eyes to follow user moving) | 24: Image display | 10 s/cycle | Pay attention (Worry or care) |
| a25 | Change facial expression 7 (Display letter or symbol on eyes) | 24: Text display | 3 s/cycle | Show reaction (Reaction to user's emotion) |
| a26 | Tilt one's head | 35: Teaching command Rotation speed 200 mm/s | 2 s/cycle | Show reaction (Show that one cannot understand the user's emotion) |
| a27 | React when eye contact is made (Perform any of the actions when eye contact is made) | Depending on the action performed | Depending on the action performed | Show reaction |
| an | ... | ... | ... | ... |

The numbers in the command content in Tables 2A and 2B represent the codes of control targets. "Teaching command" is an operation command taught in advance using a teaching method, such as offline teaching, online teaching, or direct teaching. "Tracking command" is an operation command for tracking the position and posture of the user 200 based on various sensor information such as a captured image Im (distance image).

The motor controller 113 controls the drive of the servomotor 35 in response to a command to perform the action at of the robot 100 from the action controller 112. In the case where the action content of the robot 100 is, for example, "hug", the motor controller 113 controls the position and posture of the robot 100 relative to the position and posture of the user 200 by using the tracking command, and then executes the operation command of "hug", which is taught in advance.

The signal outputter 114 controls communication between the controller 13 and the display 24 in response to a command from the action controller 112 to the robot 100 to perform the action at. In the case where the action content of the robot 100 is, for example, "smile with eyes", the signal outputter 114 outputs a command to display an image of a smile, to the right eye display 24a and the left eye display 24b.

The signal outputter 114 controls communication between the controller 13 and the speaker 25 in response to a command from the action controller 112 to the robot 100 to perform the action at. In the case where the action content of the robot 100 is, for example, "speak aloud (call)", the signal outputter 114 outputs a voice output signal to the speaker 25.

Furthermore, the signal outputter 114 controls communication between the controller 13 and the light 26 in response to a command from the action controller 112 to the robot 100 to perform the action at. In the case where the action content of the robot 100 is, for example, "make the cheek lights blink" the signal outputter 114 outputs an on-off signal to the switching elements of the right cheek light 26a and the left cheek light 26b.

<Configuration of Learning Device 300>

(Example of Hardware Configuration of Learning Device 300)

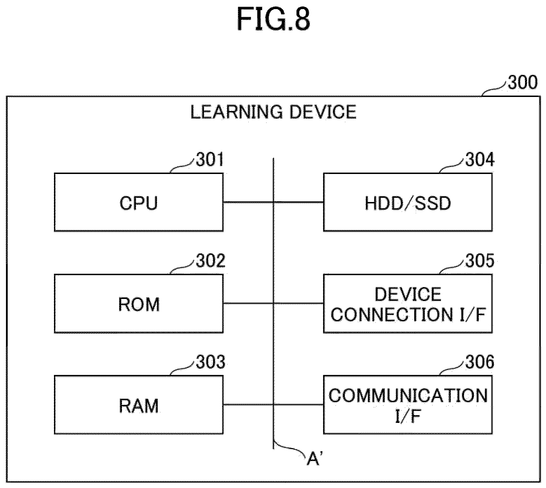

FIG. 8 is a block diagram illustrating an example of the hardware configuration of the learning device 300. FIG. 8

15

illustrates an example in which the first estimator 107, the second estimator 108, the selector 109, the information outputter 110, and the training part 124 illustrated in FIG. 7 are configured as a learning device 300 communicably connected to the robot 100. However, the functions of the first estimator 107, the second estimator 108, the selector 109, the information outputter 110, and the training part 124 may be provided inside the robot 100 as illustrated in FIG. 7.

The learning device 300 is constructed by a computer and includes a CPU 301, a ROM 302, a RAM 303, an HDD/SSD 304, a device connection I/F 305, and a communication I/F 306. These are connected to each other through a system bus A' so that they can communicate with each other. In order to improve the training processing capability of the computer, it is preferable that the learning device 300 has a GPU (graphics processing unit) or a PC cluster or the like having a plurality of computers.

The CPU 301 executes control processing including various arithmetic processing. The ROM 302 stores programs used to drive the CPU 301, such as IPL. The RAM 303 is used as a work area of the CPU 301. The HDD/SSD 304 stores various information, such as programs, an image Im captured by the camera 11, the biological information B acquired by the vital sensor 14, or detection information by various sensors.

The device connection I/F 305 is an interface for connecting the first estimator 107 to various external devices. The external devices include the camera 11, the tactile sensor 12, the vital sensor 14, the first capacitance sensor 21, and the second capacitance sensor 31. However, the first estimator 107 may acquire detection information of these various sensors from the robot 100 via the communication I/F 306, which is described in the following.

The communication I/F 306 is an interface for communicating with an external device, such as the robot 100, via a communication network or the like.

For example, the first estimator 107 connects to the Internet via the communication I/F 136 and communicates with an external device via the Internet. The first estimator 107 directly performs wireless communication with external devices via the communication I/F 306.

At least a part of the function implemented by the CPU 301 may be implemented by an electric circuit or an electronic circuit.

(Function Configuration of Learning Device 300)

FIG. 9 is a block diagram illustrating an example of the function configuration of the learning device 300. The learning device 300 includes the first estimator 107, the second estimator 108, the selector 109, the information outputter 110, the training part 124, a result acquirer 123, and a communication controller 125. In the case where the function of the learning device 300 is provided inside the robot 100, the communication controller 125 and a storage 126 become unnecessary. In the case where the learning device 300 estimates an action at of the robot 100 suitable for psychological state information st of the user 200 using the trained model LM or based on a predetermined algorithm, the result acquirer 123 and the training part 124 become unnecessary. In the following description, descriptions of the above-described structures already described in FIG. 7, etc. will be omitted as appropriate.

The functions of the action determiner 122 and the result acquirer 123 can be realized by a processor, such as the CPU 301 executing a process specified by a program stored in nonvolatile memory such as the ROM 302 or the like. The function of the communication controller 125 can be realized by the communication I/F 306 or the like. Furthermore, the function of the storage 126 can be realized by a nonvolatile memory such as the HDD/SSD 304.

The learning device 300 according to the present embodiment estimates a psychological state of the user 200, performs reinforcement learning, and estimates an action at of the robot 100 suitable for psychological state information st of the user 200. As the reinforcement learning algorithm, any one of Q-learning, SARSA, Monte Carlo method, and deep reinforcement learning (reinforcement learning using DQN (Deep-Q-Network)) can be used. In the following, Q-learning and deep reinforcement learning will be described with examples.

The first estimator 107 executes various processes for estimating a psychological state of the user 200. The first estimator 107 estimates a psychological state of the user 200 based on a captured image Im of the user 200, and outputs the obtained first psychological state information sat of the user 200. The captured image Im includes at least a face image of the user 200. The first psychological state information sat of the user 200 is a predetermined state classified based on a face image of the user 200.

The first estimator 107 has a first psychological state estimator 151. The first psychological state estimator 151 is responsible for one process in the first estimator 107. Another external device communicably connected to the learning device 300 may be responsible for the function of the first psychological state estimator 151. The first psychological state estimator 151 estimates a psychological state of the user 200 based on a face image of the user 200. The psychological state of the user 200 is classified into predetermined states based on a face image of the user 200. For example, a psychological state of the user 200 is preferably classified into at least one of "neutral", "happiness", "sadness", "disgust", "fear", "surprise", or "anger". A psychological state of the user 200 may be a combination of "fear" and "surprise", for example.

The first psychological state estimator 151 estimates a psychological state using a trained model or while performing machine learning. For example, the first psychological state estimator 151 uses training data to learn a trained model of a neural network to which a face image of the user 200 is input and which outputs a psychological state of the user 200 including "neutral", "happiness", "sadness", and "disgust". Thus, when a new face image of the smiling user 200 is input to the trained model of the neural network, an AI (artificial intelligence) system can analyze it and classify a psychological state of the user 200 as "happiness".

The second estimator 108 executes various processes for estimating a psychological state of the user 200. The second estimator 108 estimates a psychological state of the user 200 based on at least one of heart rate, respiration rate, blood pressure value, or body temperature of the user 200, and outputs the obtained second psychological state information sbt. The second psychological state information sbt of the user 200 is a predetermined state classified based on information related to at least one of heart rate, respiration, blood pressure, or body temperature of the user 200.

The second estimator 108 has a second psychological state estimator 152. The second psychological state estimator 152 is responsible for one process in the second estimator 108. Another external device communicably connected to the learning device 300 may be responsible for the function of the second psychological state estimator 152. The second psychological state estimator 152 estimates a psychological state of the user 200 based on information about at least one of heart rate, respiration rate, blood pressure, or body temperature of the user 200. The psychological state of the user 200 is classified into a predetermined state based on information related to at least one of heart rate, respiration rate, blood pressure, or body temperature of the user 200. For example, a psychological state of the user 200 is preferably classified into at least one of "neutral", "happiness", "sadness", "disgust", "fear", "surprise", or "anger". A psychological state of the user 200 may be a combination of "fear" and "surprise", for example.

The second psychological state estimator 152 estimates a psychological state using a trained model or while performing machine learning. For example, the second psychological state estimator 152 uses training data to train a model of a neural network to which information related to at least one of heart rate, respiration rate, blood pressure, or body temperature of the user 200 is input and which outputs emotions of the user 200 including "neutral", "happiness", "sadness", and "disgust". Thus, when information related to at least one of new heart rate, respiration rate, blood pressure, or body temperature when the user 200 is smiling is input to the trained model of the neural network and AI (artificial intelligence) analysis is performed, the psychological state of the user 200 can be classified as "happiness".

The result acquirer 123 acquires information related to an evaluation of a user's response as a result of the action at of the robot 100. The information related to the response evaluation preferably includes information related to an emotion of the user 200. The information related to an emotion of the user 200 includes at least a positive or negative emotion level of the user 200.

The result acquirer 123 has a psychological state level estimator 153. The function of the psychological state level estimator 153 may be performed by another external device communicably connected to the learning device 300. The function of the psychological state level estimator 153 may be performed by at least one of the first psychological state estimator 151 or the second psychological state estimator 152. The psychological state level estimator 153 estimates a psychological state level of the user 200 based on a captured image Im (face image) of the user 200 or information related to at least one of heart rate, respiration rate, blood pressure, or body temperature of the user 200.

For example, a psychological state of the user 200 is classified into a predetermined state based on a face image of the user 200 or information related to at least one of heart rate, respiration rate, blood pressure, or body temperature of the user 200. For example, an emotion of the user 200 is preferably classified into at least one of "neutral", "happiness", "sadness", "disgust", "fear", "surprise", or "anger" An emotion of the user 200 may be a combination of "fear" and "surprise", for example.

The psychological state level estimator 153 estimates a psychological state using a trained model or while performing machine learning. For example, the psychological state level estimator 153 uses training data to train a model of a neural network to which a face image of the user 200 is input and which outputs emotions of the user 200 including "neutral", "happiness", "sadness", and "disgust". Thus, when a new face image of the smiling user 200 or information related to at least one of heart rate, respiration rate, blood pressure, or body temperature is input to the trained model of the neural network, an AI (artificial intelligence) system can analyze it and classify a psychological state of the user 200 as "happiness".

When a psychological state of the user 200 is classified as "neutral", the psychological state level estimator 153 estimates a psychological state level as "neutral". when a psychological state is classified as "happiness", the psychological state level estimator 153 estimates a psychological state level as "very positive". When a psychological state of the user 200 is classified as "sadness", the psychological state level estimator 153 estimates a psychological state level as "negative". When the psychological state is classified as "disgust", the psychological state level estimator 153 estimates a psychological state level as "very negative".

Thus, the result acquirer 123 acquires a response evaluation of the user's response (for example, the emotion level of the user 200) as a result of the action at of the robot 100.

The training part 124 generates a trained model LM to which psychological state information st of the user 200 is input and which outputs a value $Q(st,at)$ of an action at of the robot 100 by reinforcement learning. The training part 124 also updates the trained model LM based on the response evaluation of user's response. In the present embodiment, the training part 124 acquires a reward r for an action at of the robot 100 based on the response evaluation of a user's response, and updates a value Q (action value table TB3) of the action at for psychological state information st of the user 200 based on the reward r.

The training part 124 has a reward acquirer 155, a value updater 156, the storage 126, and an action determiner 122. The reward acquirer 155 acquires a reward r for an action at of the robot 100 based on a response evaluation (for example, a psychological state level of the user 200).

The storage 126 stores information related to a predetermined reward rn (n is an identification number of a reward r) based on the response evaluation. The information related to the reward rn is managed, for example, by a table in the database. Table 3 below is an example of the reward table TB4 showing the predetermined reward rn. The reward table TB4 has reward IDs for identifying each reward rn, a response evaluation of a user's response, and the reward rn based on the response evaluation. The number of rewards rn is as large as the number of predefined results of a response evaluation of a user's response.

[Table 3]

| Reward ID | Response Evaluation of User's Response | Reward |
|---|---|---|
| r0 | Emotion level: Neutral | 0 |
| r1 | Emotion level: Slightly positive | +1 |
| r2 | Emotion level: Positive | +2 |
| r3 | Emotion level: Very positive | +3 |
| r4 | Emotion level: Slightly negative | -1 |
| r5 | Emotion level: Negative | -2 |
| r6 | Emotion level: Very negative | -3 |
| rn | ... | ... |

The reward acquirer 155 acquires a predetermined reward rn corresponding to a psychological state level of the user 200. Preferably, the more positive the psychological state level of the user 200 is, the more positive the reward rn is defined. It is preferable that the more negative the psychological state level of the user 200 is, the more negative the reward rn is defined. On the other hand, when the psychological state level of the user 200 is "neutral", the reward acquirer 155 acquires a reward rn of 0.

The value updater 156 updates the value Q of an action at of the robot 100 corresponding to psychological state information st of the user 200, based on the predetermined reward rn. In Q-learning, the value Q is updated by the following Expression 1.

[Expression 1]

$$Q(st, at) = Q(st, at) + \alpha \left( r + \gamma \max Q(st+1, at+1) - Q(st, at) \right) \quad \cdots \text{Expression 1}$$

In Expression 1, st is a psychological state of the user 200 at a certain time t, and at is an action of the robot 100 at a certain time t. An action at of the robot 100 changes a psychological state of the user 200 to st+1 (t+1 is a next time step). r is the reward obtained by the change in the psychological state. The term with "max" is the value Q obtained by multiplying the discount rate $\gamma$ $(0 < \gamma \leqq 1)$ by the value Q obtained in the case where the action at+1 with a highest value known at that time is selected for the psychological state st+1. $\alpha$ is the learning coefficient $(0 < \gamma \leqq 1)$, which adjusts the learning rate.

Expression 1 shows a method for updating the value Q(st,at) of an action at for psychological state information st of the user 200 based on the reward r returned as the result of the action at of the robot 100. In the case where the value Q of an action at of the robot 100 for psychological state information st of the user 200 is smaller than the sum of the reward r and the discounted value Q of the best action at+1 for next psychological state information st+1, the value Q(st,at) is increased. In contrast, in the case where the value of an action at of the robot 100 for psychological state information st of the user 200 is larger than the sum of the reward r and the discounted value Q of the best action at+1 for next psychological state information st+1, the value Q(st,at) is decreased. Therefore, Expression 1 makes the value Q of an action at for psychological state information st close to the sum of the resultant reward r and the discounted value Q of the best action at+1 for next psychological state information st+1.

The value updater 156 updates the value Q(sn, an) of the action value table TB3 according to Expression 1. The state observer 121 observes next psychological state information st+1 of the user 200. The action determiner 122 determines an action at+1 of the robot 100 for next psychological state information st+1 of the user 200 by the ε-greedy method or the like based on the value Q of the action at (the action value table TB3 in this example).

The storage 126 stores information related to a psychological state sn of the user 200 (n is an identification number of a state s). The information related to a psychological state sn is managed, for example, by a table in the database. Table 4 below is an example of the state table TB2 related to a psychological state sn of the user 200. The state table TB2 has a state ID for identifying a psychological state sn of the user 200 and the content of the psychological state of the user 200. Each psychological state sn of the user 200 corresponds to one of the predefined types of the emotions of the user 200. In the case where the function of the learning device 300 is provided inside the robot 100, the storage 103 of the robot 100 stores the state table TB2.

[Table 4]

| State ID | State |
|----------|-------|
| s0 | Emotion: Neutral |
| s1 | Emotion: Happiness |
| s2 | Emotion: Sadness |
| s3 | Emotion: Disgust |
| s4 | Emotion: Fear |
| s5 | Emotion: Surprise |
| s6 | Emotion: Anger |
| sn | ... |

For example, in the case where a psychological state information st of the user 200 is "sadness", it is observed that the user 200 is depressed. Therefore, when the robot 100 performs the action at of "push", for example, the user 200 may feel uncomfortable in communication. In the case where the user 200 has a negative feeling, the user 200 becomes bored with and indifferent to the robot 100. On the other hand, when the robot 100 performs an action at of cheering or reassuring the user 200, such as "tapping" the user 200, it is more likely to elicit a favorable response from the user 200.

In the case where a psychological state information st of the user 200 is "fear", the user 200 is observed to be in a state of being frightened or anxious about something. Therefore, when the robot 100 performs the action at of "smile with eyes", for example, the user 200 may feel uncomfortable in communication. In the case where the user 200 has a negative feeling, the user 200 becomes bored with and indifferent to the robot 100. On the other hand, when the robot 100 performs an action at of reassuring the user 200, such as "holding a hand" of the user 200, it is more likely to elicit a favorable response from the user 200. Thus, an inference is made about a certain correlation between the psychological state information st of the user 200 and the value Q of the action at of the robot 100.

The action determiner 122 determines an action at of the robot 100 corresponding to psychological state information st of the user 200 based on the value Q of the action at-1 (t-1 is a previous time step). The storage 126 stores an action value table TB3 representing the value Q of an action an of the robot corresponding to a psychological state sn of the user 200. The following Table 5 shows an example of the action value table TB3 at a certain time t.

[Table 5]

|     | a0  | a1  | a2  | a3  | a4  | a5  | a6  | a7  | a8  | a9  | a10 | an  |
|-----|-----|-----|-----|-----|-----|-----|-----|-----|-----|-----|-----|-----|
| s0  | +1  | +1  | +1  | 0   | 0   | -1  | +1  | 0   | -1  | +1  | 0   | ... |
| s1  | +2  | +1  | +1  | +1  | +1  | 0   | +2  | +1  | +2  | +1  | +1  | ... |
| s2  | +3  | +3  | +4  | -2  | -1  | -2  | +3  | +1  | -1  | +1  | -1  | ... |
| s3  | -1  | 0   | -1  | -1  | -1  | -1  | 0   | 0   | -1  | -1  | 0   | ... |
| s4  | 0   | -1  | 0   | -1  | -1  | -2  | +2  | 0   | -1  | 0   | -1  | ... |
| s5  | 0   | 0   | -1  | -2  | -1  | 0   | 0   | 0   | 0   | -1  | -1  | ... |
| s6  | -2  | -1  | -1  | -3  | -2  | -3  | 0   | 0   | -1  | -2  | -2  | ... |
| sn  | ... | ... | ... | ... | ... | ... | ... | ... | ... | ... | ... | ... |

In an initial state (time t = 0, etc.) of the action value table TB3, the value Q of an action at of the robot 100 corresponding to psychological state information st of the user 200 is not known. Therefore, it is preferable that the action determiner 122 initializes the value Q of all actions an by a random number and selects one action at from predetermined actions an.

If the action determiner 122 continues to select only an action at with the highest value Q and continues with training, there will be no chance of transition to psychological state information st+1 (t+1 is a next time step) that has not yet been experienced. It is therefore preferable that the action determiner 122 use the ε-greedy method or the like to select an action at with a highest value Q with a probability of 1-ε and select one action at from all actions an with a probability of ε.

For example, if psychological state information st of the user 200 is "sadness", the action determiner 122 selects the "tapping" action a4 with the highest value Q with a probability of 0.9 (ε = 0.1). This increases the possibility that the user 200 will respond favorably. The action determiner 122 freely selects an action at from all actions an with a probability of 0.1. This allows the user 200 to feel that the robot 100 is selecting an action at under free will and maintain their interest in the robot 100. If there is no highest value Q but there are multiple values of the same value Q, the action determiner 122 selects one action at from the highest value Q in the same column by a random number.

The communication controller 125 transmits to the robot 100 a command to perform the action at determined by the action determiner 122. The robot 100 receives a command to perform the action at from the communication controller 102. Subsequently, the action controller 112 commands the motor controller 113 or the signal outputter 114 to perform the action at of the robot 100. Thus, the robot 100 performs the action at that elicits a favorable response of the user 200 in accordance with the psychological state information st of the user 200.

The communication controller 125 transmits to the robot 100 a command to perform a determined action at+1. The robot 100 receives a command to perform the action at+1 from the learning device 300 via the communication controller 102. Subsequently, the action controller 112 commands the motor controller 113 or the signal outputter 114 to execute the action at+1 of the robot 100. The robot 100 thereby performs the action at+1 suitable for the psychological state information st+1 of the user 200.

Herein, as a method of expressing the value Q(st,at) on a computer, as described above, there is a method of storing the value Q(st,at) as the action value table TB3 for combinations of all psychological states sn of the user 200 and all actions an of the robot 100. Another method is to prepare an action value function that approximates the action value table TB3. The latter method can be realized by adjusting the parameters of the approximation function by a method such as stochastic gradient descent. For example, as an approximation function, the training part 124 preferably generates a trained model of a neural network (DQN) to which psychological state information st of the user 200 is input and which outputs the value Q of an action at of the robot 100 by deep reinforcement learning.

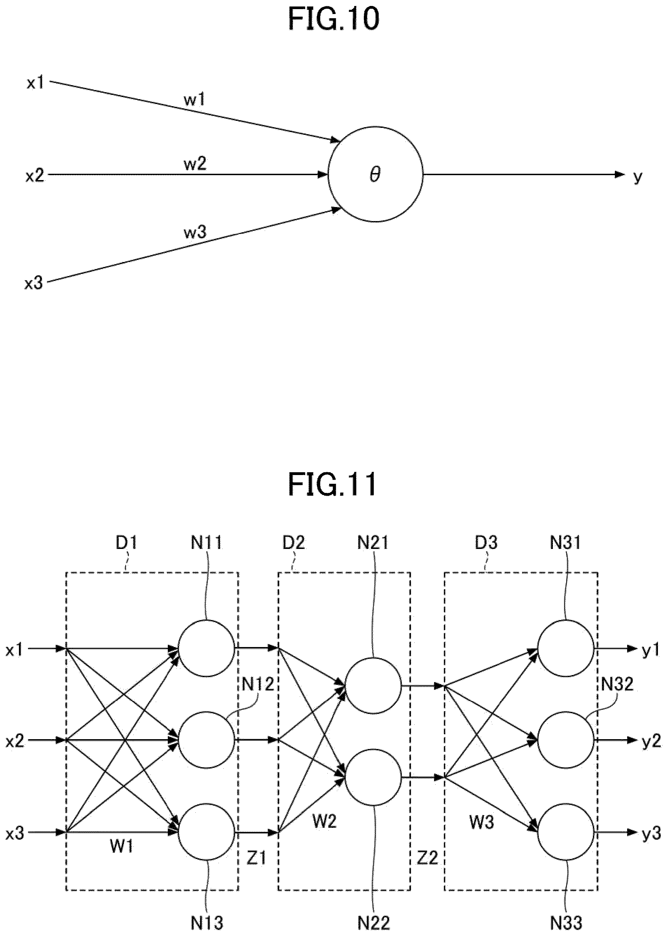

The deep reinforcement learning will be described below, but first, a neural network will be described. FIG. 10 schematically illustrates a neuron trained model, and FIG. 11 schematically illustrates a trained model of a three-layer neural network configured by combining the neuron illustrated in FIG. 10. The neural network is composed of, for example, an arithmetic unit and a memory simulating a model of a neuron (simple perceptron) illustrated in FIG. 10.

As illustrated in FIG. 10, the neuron outputs an output (result) y for a plurality of inputs x (input x1 through input x3 in FIG. 10). Each input x (x1, x2, x3) is multiplied by a weight w (w1, w2, w3) corresponding to the input x. Thus, the neuron outputs an output y expressed by the following Expression 2. Note that input x, output y, and weight w are all vectors. In Expression 2 below, θ is a bias and fk is an activation function.

[Expression 2]

$$y = f_k \left( \sum_{i=1}^{n} x_i w_i - \theta \right) \quad \cdots \text{Expression 2}$$

FIG. 11 illustrates a three-layer neural network composed of the neuron illustrated in FIG. 10. As illustrated in FIG. 11, a plurality of inputs x (input x1 through input x3 for example) are input from the left side of the neural network, and a result y (output y1 through output y3 for example) is output from the right side. Specifically, inputs x1, x2, and x3 are input by multiplying each of three neurons N11 through N13 with corresponding weights. The weights assigned to these inputs are collectively denoted as "W1".

Neurons N11 through N13 output z11 through z13, respectively. In FIG. 11, these z11 through z13 are collectively denoted as "feature vector Z1", and can be regarded as vectors from which the feature values of the input vectors are extracted. This feature vector Z1 is a feature vector between weights W1 and W2. z11 through z13 are input by multiplying each of two neurons N21 and N22 with corresponding weights. The weights assigned to these inputs are collectively denoted as "W2".

Neurons N21 and N22 output z21 and z22, respectively. In FIG. 11, z21 and z22 are collectively denoted as a feature vector "Z2". This feature vector Z2 is a feature vector between weights W2 and W3. z21 and z22 are input by multiplying each of three neurons N31 through N33 with corresponding weights. The weights assigned to these inputs are collectively denoted as "W3".

Lastly, neurons N31 through N33 output y1 through y3, respectively. The operation of the neural network includes a training mode in which weights W1 through W3 of the neural network are learned, and an estimation mode in which outputs y1 through y3 are estimated from inputs x1 through x3. For example, in the training mode, weights W1 through W3 are learned using the training data set, and the action at of the robot 100 is determined in the estimation mode using the parameters. Although the term "estimation" is used herein for convenience, it is to be understood that various tasks, such as detection and classification, may also be performed.

Weights W1 through W3 can be learned by backpropagation. Error information enters from the right side of the neural network and flows to the left side. The backpropagation adjusts (learns) the weights of each neuron so as to reduce the difference (error) between output y when input x is input and true output y (label data).

Such neural networks can perform deep learning with three or more layers. It is possible to automatically acquire an arithmetic unit having a convolutional neural network (CNN) that extracts input features stepwise and a neural network that classifies or regresses outputs only from the training data.

In the action value table TB3 described above, the memory space may become too large when the number of psychological states sn of the user 200 and the number of actions an of the robot 100 become large. Accordingly, the memory space of the action value table TB3 can be prevented from increasing by approximating the action value table TB3 by a function using a neural network (DQN).

Referring again to FIG. 9, the configuration of the first estimator 107 for performing deep reinforcement learning will be described. The training part 124 has two neural networks (DQN) including a target network TN (value $Q(st,at)|\theta^-$) and a Q network QN (value $Q(st,at)|\theta$). The two networks are stored in the storage 126. The structure of the two networks is the same, but the parameter $\theta$ (corresponding to the weight described above) is different. The inputs of the two networks are psychological state information st of the user 200, and the output is the value $Q(st,at)$ of an action at of the robot 100.

The selector 109 outputs psychological state information st of the user 200 to the action determiner 122 and the training part 124. The action determiner 122 inputs the psychological state information st of the user 200 to the target network TN, and determines an action at of the robot 100 by the $\varepsilon$-greedy method or the like based on the value $Q(st,at|\theta^-)$ of the action at output from the target network TN. The communication controller 125 transmits a command to perform the determined action at to the robot 100, and the robot 100 performs the action at in response to the command to perform the action at.

The result acquirer 123 acquires a response evaluation of the user's response (an emotion level of the user 200) as a result of the action at of the robot 100, and outputs the response evaluation to the training part 124. The training part 124 acquires a reward r based on the response evaluation. The first estimator 107 acquires first psychological state information sat+1 of the next user 200. The second estimator 108 acquires second psychological state information sbt+1 of the next user 200. The selector 109 selects either the first psychological state information sat+1 or the second psychological state information sbt+1 as the psychological state information st+1 based on the selection information, and outputs it to the action determiner 122 and the training part 124 via the information outputter 110.

The training part 124 stores an experience et (< st, at, st+1, r >) of the robot 100 in the storage 126 as an experience buffer. Herein, st is a psychological state of the user 200, at is an action of the robot 100, st+1 is a next psychological state of the user 200, and r is a reward. It is preferable that the training part 124 clip the reward r within the range of -1 to +1 so as not to excessively react to outliers or the like (so-called reward clipping).

The training part 124 periodically acquires an optional experience et from the storage 103 (experience buffer) to train the Q network QN. For example, the training part 124 acquires an experience (B is e0 through en) for mini-batch learning B from the storage 126. Subsequently, the training part 124 updates the parameter $\theta$ of the Q network QN so as to minimize a TD (temporal difference) error $L(\theta)$ shown in Expression 3 below (so-called experience replay).

[Expression 3]

$$L(\theta) = \frac{1}{|B|} \sum_{e \in B} (r + \gamma \max Q(st+1, at+1 \mid \theta^-) - Q(st, at \mid \theta))^2 \quad \cdots \text{Expression 3}$$

Next, the training part 124 reflects the parameter $\theta$ of the Q network QN to the target network TN at a discretionarily determined interval. The training part 124 may periodically copy all the parameters $\theta$ of the Q network QN to the target network TN, or may reflect the parameter $\theta$ of the Q network QN little by little each time the parameter $\theta$ of the Q network QN is updated.

The action determiner 122 inputs next psychological state information st+1 of the user 200 to the target network TN. The action determiner 122 determines an action at+1 of the robot 100 by the $\varepsilon$-greedy method or the like based on the value $Q(st+1, at+1|\theta^-)$ of the action at+1 that is output from the target network TN. The communication controller 125 transmits a command to perform the determined action at+1 to the robot 100, and the robot 100 performs the action at+1 in response to the command to perform the action at+1.

As described above, the first estimator 107 can perform deep reinforcement learning to estimate an action at of the robot 100 suitable for psychological state information st of the user 200.

<Example of Processing in Controller 13>

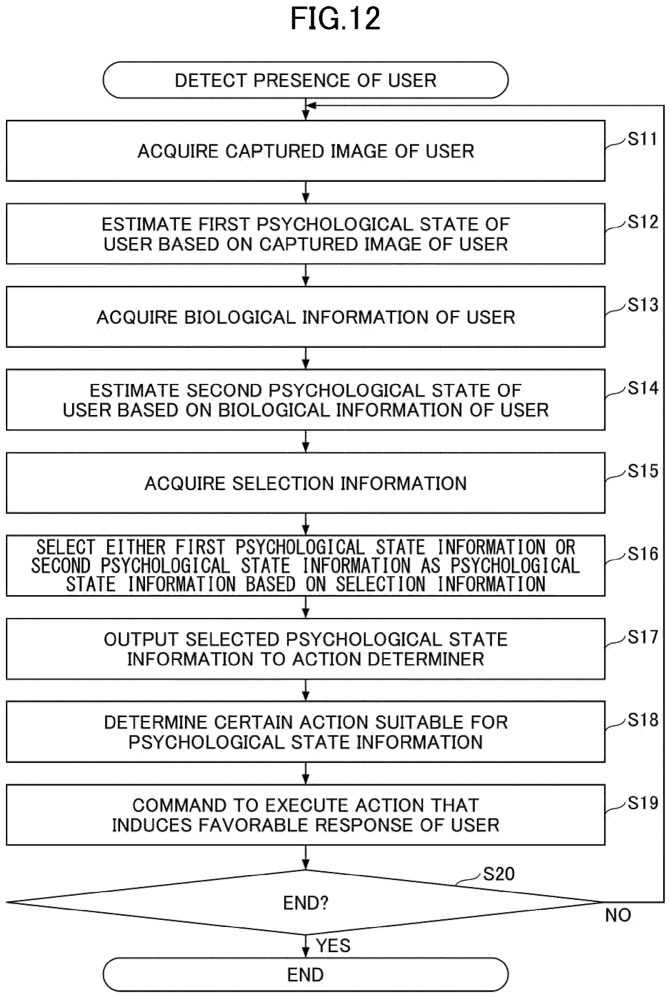

FIG. 12 is a flowchart showing processing in the controller 13 according to the first embodiment. FIG. 12 shows processing in which the controller 13 commands execution of an action at that elicits a favorable response of the user 200 corresponding to psychological state information st of the user 200. The controller 13 starts the processing of FIG. 12 when the presence of the user 200 is detected by the detector 106 based on a captured image Im from the camera 11. The detection of the presence of the user 200 means detection of the user 200 present at a position far from, close to, or in contact with the robot 100.

First, in step S11, the controller 13 causes the acquirer 101 to acquire a captured image Im of the user 200. The captured image Im includes at least a face image of the user 200. In step S11, power is supplied from the battery 15 to the camera 11, the tactile sensor 12, the first capacitance sensor 21, the second capacitance sensor 31, and the vital sensor 14. However, in order to reduce power consumption of the battery 15, power need not be supplied to the servomotor 35, the display 24, the speaker 25, and the light 26.

Subsequently, in step S12, the controller 13 causes the first estimator 107 to estimate first psychological state information sat of the user 200 based on the captured image Im. It is preferable that the controller 13 causes the first estimator 107 to estimate the first psychological state information sat of the user 200 while performing reinforcement learning or by using a trained model. The processing in step S12 is not essential to the controller 13, and can be performed by an external device communicably connected to the robot 100.

Subsequently, in step S13, the controller 13 causes the acquirer 101 to acquire biological information B of the user 200. The biological information B includes information related to at least one of heartbeat, respiration, blood pressure, or body temperature of the user 200. In step S13, power is supplied from the battery 15 to the camera 11, the tactile sensor 12, the first capacitance sensor 21, the second capacitance sensor 31, and the vital sensor 14. However, in order to reduce power consumption of the battery 15, power need not be supplied to the servomotor 35, the display 24, the speaker 25, and the light 26.

Subsequently, in step S14, the controller 13 causes the second estimator 108 to estimate second psychological state information sbt of the user 200 based on the biological information B. It is preferable that the controller 13 cause the second estimator 108 to estimate the second psychological state information sbt of the user 200 while performing reinforcement learning or by using a trained model. The processing in step S14 is not essential to the controller 13, and can be performed by an external device communicably connected to the robot 100.

Subsequently, in step S15, the controller 13 causes the acquirer 101 to acquire selection information. In the case where the image Im is captured in step S11 or the biological information B acquired in step S13 is used as the selection information, step S15 can be omitted.

Subsequently, in step S16, the controller 13 causes the selector 109 to select either the first psychological state information sa or the second psychological state information sb as psychological state information st of the user 200 based on the selection information.

The order in which the sets of steps S11 to S12, steps S13 to S14, and steps S15 to S16 are performed may be appropriately changed, or these three sets may be executed in parallel.

Subsequently, in step S17, the controller 13 causes the information outputter 110 to output the psychological state information st selected by the selector 109 to the action determiner 122.

Subsequently, in step S18, the controller 13 causes the action determiner 122 to determine a predetermined action at of the robot 100 suitable for the psychological state information st of the user 200.

Subsequently, in step S19, the controller 13 causes the action controller 112 to command to perform an action at that elicits a favorable response of the user 200 corresponding to the psychological state information st of the user 200.

Subsequently, in step S20, the controller 13 determines whether to end the processing. For example, the controller 13 determines whether to end the processing when the presence of the user 200 is no longer detected based on the captured image Im or the like, and determines whether not to end the processing when the presence of the user 200 is detected.

If it is determined in step S20 that the processing is ended (Yes in step S20), the controller 13 ends the processing. If it is determined that the processing is not ended (No in step S20), the controller 13 repeats the processing from step S11. By repeating the processing from step S11 to step S19, the controller 13 can accumulate actions at that do not cause discomfort to the user 200. The accumulation of natural actions at leads to an impression of a good partner, and communication becomes natural. Consequently, the user's engagement with the robot 100 can be sustained, allowing the robot 100 to deliver therapeutic benefits.

Thus, the controller 13 performs a processing of commanding to perform an action at that elicits a favorable response of the user 200 corresponding to psychological state information st of the user 200. In the case where the learning device 300 communicably connected to the robot 100 performs the function of the first estimator 107 in order to improve the training processing capability or to suppress the power consumption of the battery 15, the processing of step S11 is performed by the learning device 300.

At the start of the processing shown in FIG. 12, the servomotor 35, the display 24, the speaker 25, and the light 26 may be in a standby state (sleep state) in which the amount of power supplied is suppressed. In other words, the controller 13 preferably suppresses the power consumption of the battery 15 by returning various devices from the standby state in which the amount of power supplied is suppressed as necessary.

<Processing by Learning Device 300>

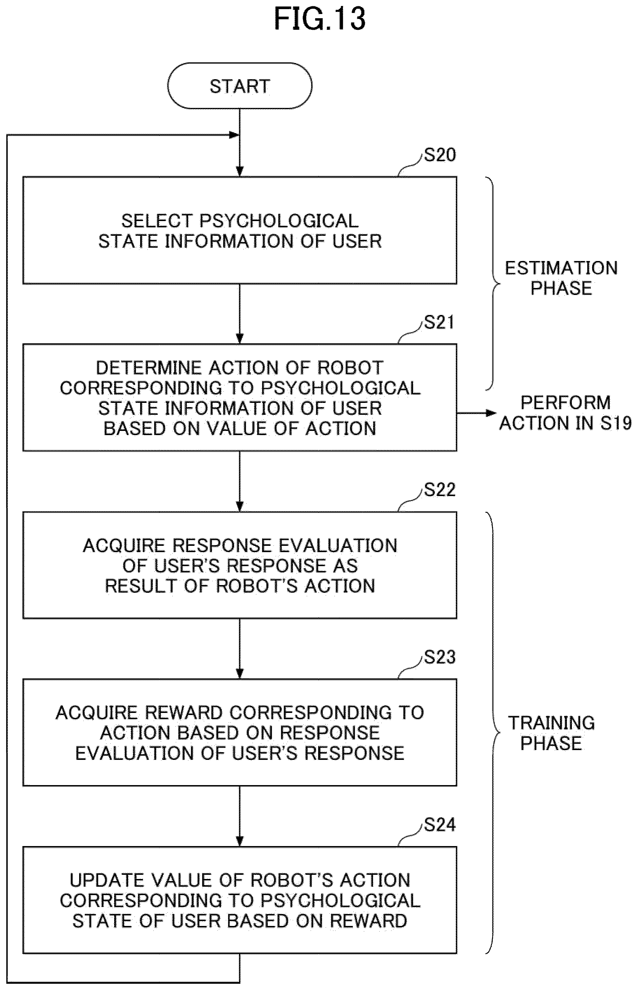

FIG. 13 is a flowchart showing the processing by the learning device 300. FIG. 13 shows the processing in which the learning device 300 performs reinforcement learning to estimate a predetermined action at of the robot 100 suitable for psychological state information st of the user 200.

First, in step S20, the learning device 300 causes the selector 109 to select either the first psychological state information sat or the second psychological state information sbt as the psychological state information st of the user 200 based on the selection information.

Next, in step S21, the learning device 300 causes the action determiner 122 to determine an action at of the robot 100 suitable for the psychological state information st of the user 200 based on the value Q of the action at-1 (the trained model LM such as the action value table TB3 or DQN described above). The learning device 300 outputs a command to perform the action at to the action controller 112 or transmits the command to the robot 100 via the communication controller 125, thereby the robot 100 performs the action at (step S19).

Steps S20 and S21 are the estimation phase for estimating an action at of the robot 100 suitable for the psychological state information st of the user 200, and the other steps are the training phase.

In step S22, the learning device 300 causes the result acquirer 123 to acquire information related to a response evaluation of a user's response as a result of the action at of the robot 100 (for example, a psychological state level of the user 200).

In step S23, the learning device 300 causes the reward acquirer 155 to acquire a reward r for the action at of the robot 100 based on the response evaluation.

In step S24, the learning device 300 causes the value updater 156 to update the value Q of the action at of the robot 100 corresponding to the psychological state information st of the user 200 based on the reward r.

After the training, the learning device 300 returns to step S20 and causes the selector 109 to select next psychological state information st+1 of the user 200. Then, in step S21, the learning device 300 causes the action determiner 122 to determine an action at+1 of the robot 100 suitable for the next psychological state information st+1 of the user 200 based on the updated value Q of the action at. Then, the robot 100 performs the next action at+1 (step S19).

After step S24, the learning device 300 may provide a step for causing the value updater 156 to determine whether or not the value Q of the action at has converged (in other words, whether or not the training has converged). If it is determined that the training has converged, the learning device 300 does not need to execute the training phase in the subsequent processing. In other words, the learning device 300 executes only the estimation phase and estimates the action at+n of the robot 100 suitable for the psychological state information st+n of the user 200 by using the trained model LM (action value table TB3 or DQN, etc.) (t+n is a time after n time steps).

<Configuration Example of Learning Device 300 according to Modified Example>

FIG. 14 is a block diagram illustrating an example of the function configuration of the learning device 300 according to a modified example. The first estimator 107 according to the modified example is different from the functional configuration of the learning device 300 illustrated in FIG. 9 in that the former performs supervised learning to estimate an action at of the robot 100 suitable for psychological state information st of the user 200. The training part 124 includes a training data storage 157, an error calculator 158, and a trained model updater 159. Hereinafter, only the differences from the configuration of the learning device 300 illustrated in FIG. 9 will be described.

The function of the training data storage 157 can be realized by nonvolatile memory such as the HDD/SSD 304. The functions of the error calculator 158 and the trained model updater 159 can be realized by a processor, such as the CPU 301 executing a process specified by a program stored in a nonvolatile memory such as the ROM 302 or the like.

The training part 124 can use a decision tree (regression tree), neural network, logistic regression, or the like as a trained model LM for performing supervised learning. Hereinafter, an example will be described in which the training part 124 generates a trained model LM of a neural network to which psychological state information st of the user 200 is input and which outputs the value Q of an action at of the robot 100 by supervised learning.

The training data storage 157 stores training data obtained in the past by, for example, another robot 100 or simulation. The training data is data with results (labels) including psychological state information st-n of the user 200 (t-n is n steps before time t), an action at-n of the robot 100, and the value Q (corresponding to a label) of the action at-n. The learning device 300 acquires training data from another robot 100 or another external device via the communication controller 125 or the like. The learning device 300 may also store the experience experienced by the robot 100 itself as training data.

The error calculator 158 first acquires the training data from the training data storage 157, and calculates an error L of the value Q of the action at based on the training data. For example, in the case where an actual response of the user 200 is favorable, the error calculator 158 assumes that there is an error of -log(Q(st,at)) and calculates an error L. In the case where an actual response of the user 200 is not favorable, the error calculator 158 assumes that there is an error of -log(1-Q(st,at)) and calculates an error L.

The trained model updater 159 updates the parameters (e.g., the aforementioned weights) of the trained model LM of the neural network so as to minimize the error L. To update the trained model LM, backpropagation can be used. Thus, the training part 124 generates a trained model LM that has been trained to a certain level based on the training data.

Thereafter, the learning device 300 uses the trained model LM generated by supervised learning to estimate an action at suitable for actual psychological state information st of the user 200. Furthermore, the robot 100 performs the action at that elicits a favorable response of the user 200 in accordance with the psychological state information st of the user 200.

More specifically, the learning device 300 causes the selector 109 to select either the first psychological state information sat estimated by the first estimator 107 or the second psychological state information sbt estimated by the second estimator 108 as psychological state information st of the user 200. The action determiner 122 determines a predetermined action at suitable for the psychological state information st of the user 200 using the trained model LM. Then, the communication controller 125 transmits a command to perform the determined action at to the robot 100, and the robot 100 performs the action at in response to the command to perform the action at.

The result acquirer 123 acquires a response evaluation of a user's response as a result of the action at of the robot 100. The error calculator 158 calculates an error L of the value Q of the action at based on the response evaluation and the trained model updater 159 further updates the trained model LM of the neural network so as to minimize the error L. Then, the action determiner 122 determines a predetermined action at+1 of the robot 100 suitable for next psychological state information st+1 of the user 200 using the trained model LM.

As described above, the learning device 300 estimates an action at of the robot 100 suitable for psychological state information st of the user 200 using the trained model LM trained to a certain level by supervised learning. Thus, even if for example the robot 100 were to break down and be replaced with a robot 100 of the same model number, the replaced robot 100 could learn past experiences based on the training data of the broken-down robot 100 and immediately perform an action at suitable for psychological state information st of the user 200. Furthermore, the robot 100 can perform an action at suitable for psychological state information st of the user 200 at a certain level even for a user 200 whom the robot 100 meets for the first time.

<Example of Processing in Learning Device 300 according to Modified Example>

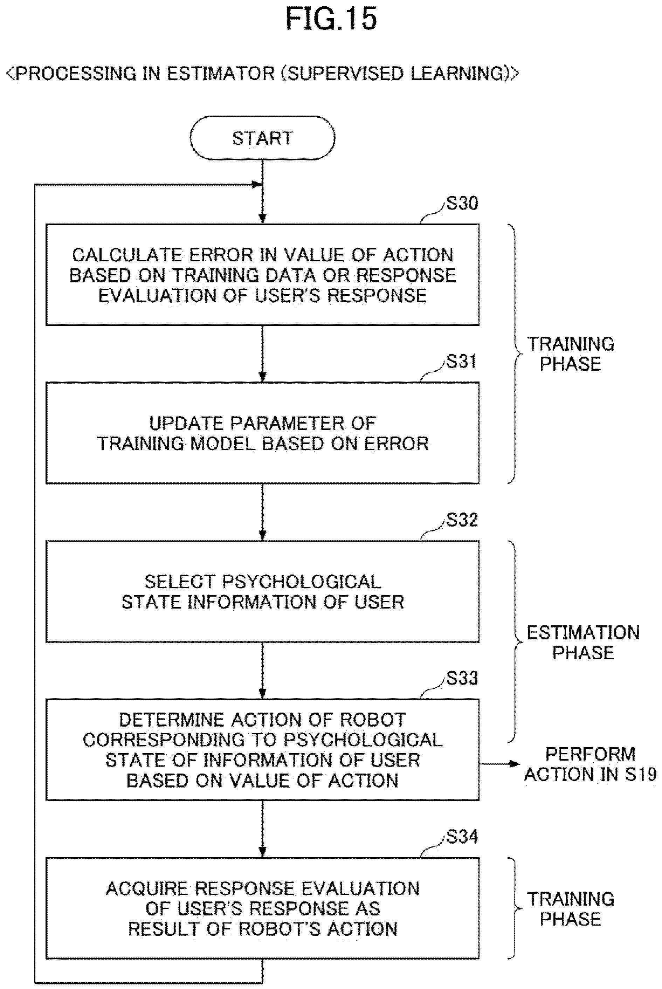

FIG. 15 is a flowchart showing the processing by the learning device 300 according to the modified example. FIG. 15 shows the processing in which the learning device 300 performs supervised learning to estimate an action at of the robot 100 suitable for psychological state information st of the user 200.

First, in step S30, the learning device 300 obtains training data from the training data storage 157 by the error calculator 158, and calculates an error L of the value Q of an action at of the robot 100 based on the training data.

Next, in step S31, the learning device 300 updates the parameters (e.g., the aforementioned weights) of the trained model LM of a neural network by the trained model updater 159 so as to minimize the error L. Thus, the learning device 300 can estimate an action at suitable for actual psychological state information st of the user 200 by using the trained model LM trained to a certain level with the training data.

Subsequently, in step S32, the learning device 300 causes the selector 109 to select either the first psychological state information sat estimated by the first estimator 107 or the second psychological state information sbt estimated by the second estimator 108 as psychological state information st of the user 200.

Next, in step S33, the learning device 300 causes the action determiner 122 to determine an action at of the robot 100 suitable for psychological state information st of the user 200 based on the value Q of the action at-1 (the trained model LM of a neural network, etc.). The learning device 300 outputs a command to perform the action at to the action controller 112 or transmits it to the robot 100 via the communication controller 125. Thus, the robot 100 performs the action at corresponding to the psychological state information st of the user 200 (step S19).

Steps S32 and S33 are the estimation phase for estimating the action at of the robot 100 suitable for the psychological state of the user 200, and the other steps are the training phase.

In step S34, the learning device 300 causes the result acquirer 123 to acquire information related to a response evaluation of a user's response as a result of the action at of the robot 100 (a psychological state level of the user 200).

Then, in step S30, the learning device 300 causes the error calculator 158 to calculate an error L of the value Q of the action at based on an response examination of a user's response.

Then, in step S31, the learning device 300 causes the trained model updater 159 to update the parameters (weight, etc.) of the trained model LM based on the error L.

After the training, in step S32, the learning device 300 causes the state observer 121 to observe next psychological state information st+1 of the user 200. Then, in step S33, the learning device 300 causes the action determiner 122 to determine an action at+1 of the robot 100 suitable for the next psychological state information st+1 of the user 200 based on the updated value Q of the action at. Then, the robot 100 performs the next action at+1 (step S19).

A step for the learning device 300 causing the trained model updater 159 to determine whether or not the value Q of the action at has converged (in other words, whether or not the training has converged) may be provided after step S31. If it is determined that the training has converged, the learning device 300 does not need to execute the training phase in the subsequent processing. In other words, the learning device 300 executes only the estimation phase and estimates an action at+n of the robot 100 suitable for the psychological state information st+n of the user 200 (t+n is n steps after time t) using the trained model LM (neural network, etc.).

<Example of Selection Method by Selector 109>

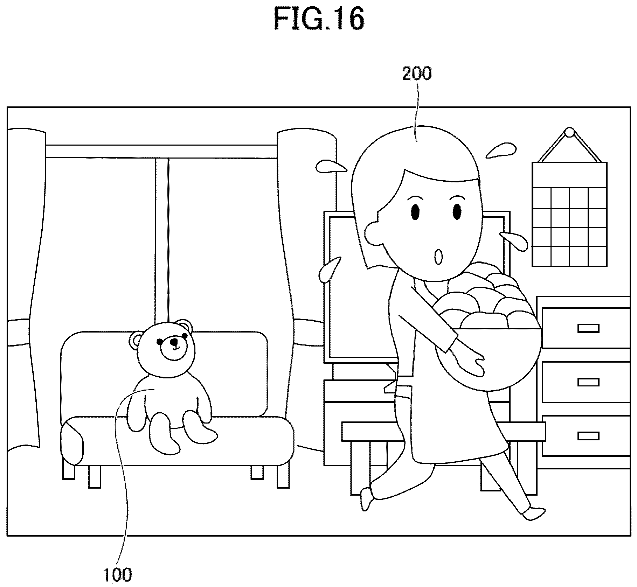

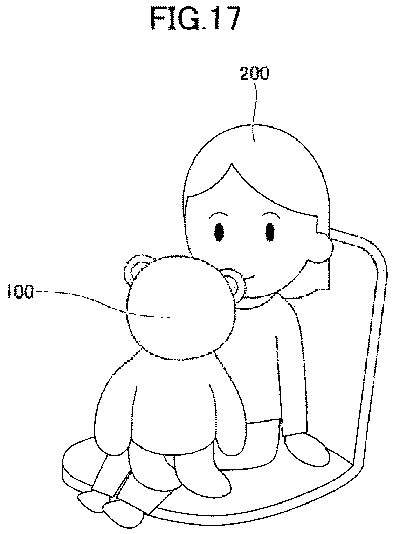

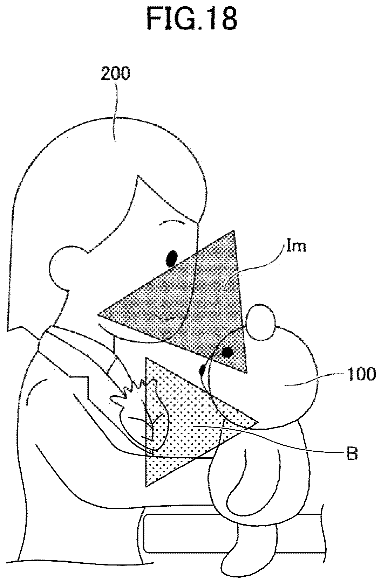

FIGS. 16 through 18 illustrate an example of the selection method by the selector 109 illustrated in FIG. 7. FIGS. 16 through 18 concretely explains the contents of Table 1. FIG. 16 is a diagram illustrating an example of a state where the user 200 is away from the robot 100. FIG. 17 is a diagram illustrating an example of a state where the face of the user 200 cannot be seen. FIG. 18 is a diagram illustrating an example of a state where the robot 100 is leaning on the user 200.

In the case where the user 200 is far away from the robot 100 as illustrated in FIG. 16, the camera 11 of the robot 100 can acquire a captured image Im that includes the user 200. The further away the user 200 is from the robot 100, the more noise tends to be added to the biological information B acquired by the vital sensor 14 of the robot 100. Therefore, when the user 200 is far away from the robot 100, it is preferable to estimate the psychological state of the user 200 using the captured image Im. For this reason, the selector 109 selects the first psychological state information sa estimated from the captured image Im. However, if the distance between the user 200 and the robot 100 becomes too long, the size of the user 200 in the captured image Im becomes small, and the accuracy of psychological state estimating decreases. For this reason, it is preferable to set conditions, such as the distance between the user 200 and the robot 100 or the size of the user 200 in the captured image Im.

As illustrated in FIG. 17, if the user 200 is near the robot 100 when holding it or performing another action that brings them close to the robot 100, the captured image Im does not include the face of the user 200, or only a part of the face of the user 200 is included in the captured image Im. Also, if the face of the user 200 does not face the camera 11, the face of the user 200 cannot be included in the captured image Im. On the other hand, as long as the vital sensor 14 faces a part of the body of the user 200, the biological information B can be acquired without a need of facing the face. Therefore, in the case where the user 200 is present near the robot 100, it is preferable to estimate a psychological state of the user 200 using the biological information B. For this reason, the selector 109 selects second psychological state information sb estimated from the biological information B.

As illustrated in FIG. 18, when the robot 100 is leaning on the user 200, only a part of the face of the user 200 is included in the captured image Im, and the accuracy of estimating a psychological state may decrease. On the other hand, the vital sensor 14 has little dependence on a relative angle between the user 200 and the robot 100. Therefore, when the robot 100 is leaning on the user 200, it is preferable to estimate a psychological state of the user 200 using the biological information B. For this reason, the selector 109 selects second psychological state information sb estimated from the biological information B.

The selection method by the selector 109 described above is only an example. The selection method by the selector 109 can appropriately adjust the selection method according to a captured image Im and the biological information B actually acquired.

<Advantageous Effects of Psychological State Information Acquisition Apparatus 160 and Robot 100>

As described above, the psychological state information acquisition apparatus 160 according to the present embodiment can estimate a psychological state of the user 200 in a multi-modal manner by using an image Im of the user 200 captured by the camera 11 and biological information B of the user 200. Thus, the present embodiment can provide the psychological state information acquisition apparatus 160 and the robot 100 with excellent accuracy in acquiring a psychological state information of the user 200.

[Second Embodiment]

Next, the second embodiment will be described. The same components as those of the foregoing embodiment will be denoted by the same reference numerals, and duplicate descriptions will be omitted as appropriate.

<Configuration Example of Controller 13 according to Second Embodiment>

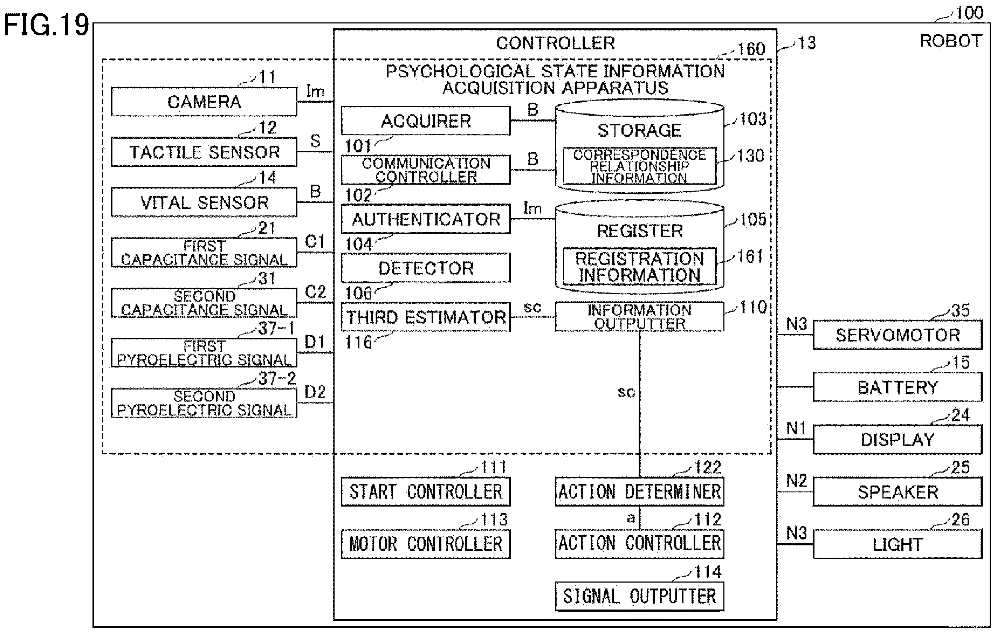

FIG. 19 is a block diagram illustrating an example of a function configuration of the controller 13 according to the second embodiment. The second embodiment is different from the first embodiment mainly in that the controller 13 has a third estimator 116 and does not have the first estimator 107, the second estimator 108, or the selector 109.

The third estimator 116 estimates a psychological state of the user 200 and outputs third psychological state information based on the trained model LM to which an image Im captured by the camera 11, biological information B acquired by the vital sensor 14, and the selection information are input and which outputs psychological state information st.

In the example illustrated in FIG. 19, the third estimator 116 estimates a psychological state of the user 200 using the trained model LM. The trained model LM is, for example, a trained action value table or a trained neural network. The third estimator 116 outputs estimated third psychological state information sc to the action determiner 122 via the information outputter 110. To estimate a psychological state of the user 200, the third estimator 116 may use not only the trained model LM, but also the trained model LM for reinforcement learning or supervised learning described in the first embodiment, or the third estimator 116 may be based on a predetermined algorithm.

The action determiner 122 estimates an action at of the robot 100 suitable for the psychological state information st of the user 200 by predetermined logic or a predetermined algorithm, based on the psychological state information st of the user 200 that is input from the third estimator 116.

<Example of Processing in Controller 13 according to Second Embodiment>

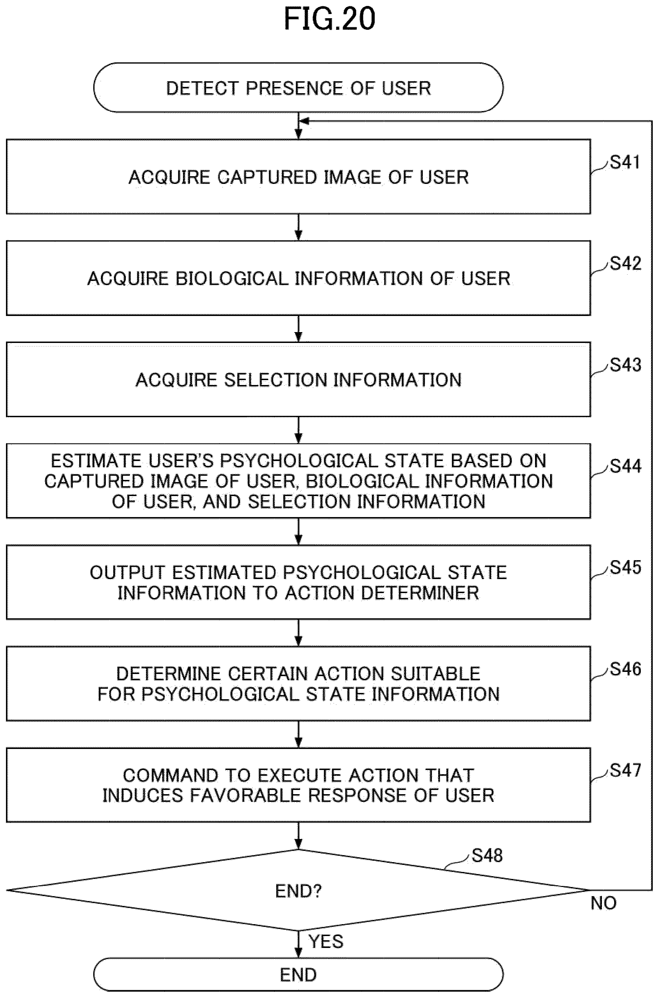

FIG. 20 is a flowchart showing an example of processing in the controller 13 according to the second embodiment. FIG. 20 shows processing in which the controller 13 commands execution of an action at that elicits a favorable response of the user 200 corresponding to psychological state information st of the user 200. The controller 13 starts the processing of FIG. 20 when the presence of the user 200 is detected by the detector 106 based on an image Im captured by the camera 11.

First, in step S41, the controller 13 causes the acquirer 101 to acquire the captured image Im of the user 200.

Subsequently, in step S42, the controller 13 causes the acquirer 101 to acquire biological information B of the user 200.

Subsequently, in step S43, the controller 13 causes the acquirer 101 to acquire selection information. In the case where the captured image Im acquired in step S41 or the biological information B acquired in step S13 is used as the selection information, step S15 can be omitted.

The processing of steps S41 through S43 may be suitably changed in order, or they may be performed in parallel.

Subsequently, in step S44, the controller 13 estimates a psychological state of the user 200 by the third estimator 116 based on the captured image Im of the user 200, the biological information B of the user 200, and the selection information.

Subsequently, in step S45, the controller 13 outputs the third psychological state information sc estimated by the third estimator 116 to the action determiner 122 by the information outputter 110.

Subsequently, in step S46, the controller 13 causes the action determiner 122 to determine a predetermined action at of the robot 100 suitable for the psychological state information st of the user 200.

Subsequently, in step S47, the controller 13 causes the action controller 112 to command to perform an action at that elicits a favorable response of the user 200 corresponding to the psychological state information st of the user 200.

Subsequently, in step S48, the controller 13 determines whether to end the processing. For example, the controller 13 determines whether to end the processing when the presence of the user 200 is no longer detected based on the captured image Im or the like, and determines whether not to end the processing when the presence of the user 200 is detected.

If it is determined in step S48 that the processing is ended (Yes in step S48), the controller 13 ends the processing. If it is determined that the processing is not ended (No in step S48), the controller 13 repeats the processing from step S41. By repeating the processing from step S41 to step S47, the controller 13 can accumulate actions that do not cause discomfort to the user 200. The accumulation of natural actions at leads to an impression of a good partner, and communication becomes natural. Consequently, the user's engagement with the robot 100 can be sustained, allowing the robot 100 to deliver therapeutic benefits.

Thus, the controller 13 of the present embodiment can perform a processing of commanding to perform an action at that elicits a favorable response of the user 200 corresponding to psychological state information st of the user 200. In the present embodiment, approximately the same advantageous effects as in the first embodiment can be obtained.

The function of the first estimator 107 of the robot 100 described above may be provided in the learning device 300 communicably connected to the robot 100 and distributed processing can be performed. Thus, the training processing capability of the computer can be enhanced. Furthermore, the distributed processing by the learning device 300 can provide technical effects, such as reduction in power consumption, reduction in the number of charging times, and reduction in the weight of the battery 15 of the robot 100.

Although the preferred embodiments have been described in detail above, they are not limited to the above-described embodiments, and various modifications and substitutions may be made to the above-described embodiments without departing from the scope of claims.

In addition, all numbers, such as ordinals and quantities used in the description of the above-described embodiments, are exemplified for the purpose of concretely explaining the technology of the present invention, and the present invention is not limited to the exemplified numbers. The connection relationships between the components are exemplified for the purpose of concretely explaining the technology of the present invention, and the connection relationships that realize the functions of the present invention are not limited thereto.

The robot according to the present embodiment is particularly suitable for applications to promote the secretion of oxytocin and provide therapeutic effects (a sense of security or self-affirmation) to a working person living alone, a senior with grown-up children living on their own, or a frail elderly person who is eligible for home health care. However, the robot is not limited to this application, and it can be used for applications to provide therapeutic effects to various users.

The psychological state information acquisition apparatus according to the present embodiment is preferably used in a robot or the like to provide therapeutic effects to a user. However, the psychological state information acquisition apparatus according to the present embodiment is not limited to robot applications, and it can be used for acquiring the psychological state of a user in a wide range of applications, such as home electric appliances and vehicles.

Aspects of the present invention are, for example, as follows.

<1> A psychological state information acquisition apparatus includes: a camera configured to capture an image of a user; a biological information acquirer configured to acquire biological information of the user by using electromagnetic waves; and an information outputter configured to output psychological state information, which is information related to a psychological state of the user obtained based on the image of the user captured by the camera and the biological information acquired by the biological information acquirer.

<2> The psychological state information acquisition apparatus described in <1> further includes: a selection information acquirer configured to acquire selection information related to at least one of a presence of motion of the user, a distance to the user, detection of a face of the user, or a relative angle with respect to the user; a first estimator configured to estimate a psychological state of the user from the captured image and output first psychological state information; a second estimator configured to estimate a psychological state of the user from the biological information and output second psychological state information; and a selector configured to consider the first psychological state information and the second psychological state information to select the psychological state information of the user based on the selection information.

<3> The psychological state information acquisition apparatus described in <2>, wherein the first estimator estimates the psychological state by a trained model to which the image captured by the camera is input and which outputs the first psychological state information, and the second estimator estimates the psychological state by a trained model to which the biological information acquired by the biological information acquirer is input and which outputs the second psychological state information.

<4> The psychological state information acquisition apparatus described in <1> further includes: a selection information acquirer configured to acquire selection information related to at least one of a presence of motion of the user, a distance to the user, detection of a face of the user, or a relative angle with respect to the user; and a third estimator configured to estimate the psychological state of the user and output third psychological state information based on a trained model to which the image captured by the camera, the biological information acquired by the biological information acquirer, and the selection information are input and which outputs the psychological state information.

<5> The psychological state information acquisition apparatus described in any one of <1> to <4>, wherein the biological information acquirer acquires the biological information by using electromagnetic waves having a frequency of 300 MHz or more and 300 GHz or less.

<6> The psychological state information acquisition apparatus described in any one of <1> to <5>, wherein the camera captures an image of the user by using light having a wavelength of 360 nm or more and 2500 nm or less.

<7> The psychological state information acquisition apparatus described in any one of <1> to <6> further includes a near-infrared light path filter for shielding visible light and transmitting near-infrared light, wherein the camera captures an image of the user using light transmitted through the near-infrared light path filter.

<8> The psychological state information acquisition apparatus described in any one of <1> to <7> further includes an authenticator configured to perform personal authentication of the user; and a controller configured to control a start of at least one of the capturing of the image by the camera and the acquiring of the biological information by the biological information acquirer based on a authentication result by the authenticator.

<9> A robot includes the psychological state information acquisition apparatus described in any one of <1> to <8>.

This application claims priority based on Japanese Patent Application No. 2023-054113, filed with the Japan Patent Office on March 29, 2023, and includes the entire contents of this Japanese Patent Application.

REFERENCE SIGNS LIST

| 1 | Torso |
|---|---|
| 2 | Head |
| 2a | Right eye |
| 2b | Left eye |
| 2c | Mouth |
| 2d | Right cheek |
| 2e | Left cheek |
| 3 | Arms |
| 3a | Right arm |
| 3b | Left arm |
| 4 | Legs |
| 4a | Right leg |
| 4b | Left leg |
| 10 | Exterior member |
| 11 | Camera |
| 12 | Tactile sensor |
| 13 | Controller |
| 14 | Vital sensor (example of biological information acquirer) |
| 141 | Microwave emitter |
| 142 | Microwave receiver |
| 15 | Battery |
| 16 | Torso frame |
| 17 | Torso mounting table |
| 21 | First capacitance sensor |
| 22 | Head frame |
| 23 | Head mounting table |
| 24 | Display |
| 24a | Right eye display |
| 24b | Left eye display |
| 25 | Speaker |
| 26 | Light |
| 26a | Right cheek light |
| 26b | Left cheek light |
| 27 | Head coupling mechanism |
| 31 | Second capacitance sensor |
| 32a | Right arm frame |
| 32b | Left arm frame |
| 33 | Right arm mounting table |
| 34a | Right arm coupling mechanism |
| 34b | Left arm coupling mechanism |
| 35 | Servomotor |
| 35a | Right arm servomotor |
| 35b | Left arm servomotor |
| 35c | Head servomotor |
| 35d | Right leg servomotor |

| 35e | Left leg servomotor |
|---|---|
| 37-1 | First pyroelectric sensor |
| 37-2 | Second pyroelectric sensor |
| 41a | Right leg wheel |
| 41b | Left leg wheel |
| 42a | Right leg frame |
| 42b | Left leg frame |
| 44a | Right leg coupling mechanism |
| 44b | Left leg coupling mechanism |
| 100 | Robot |
| 101 | Acquirer |
| 102 | Communication controller |
| 103 | Storage |
| 104 | Authenticator |
| 105 | Register |
| 106 | Detector |
| 107 | First estimator |
| 108 | Second estimator |
| 109 | Selector |
| 110 | Information outputter |
| 111 | Start controller |
| 112 | Action controller |
| 113 | Motor controller |
| 114 | Signal outputter |
| 122 | Action determiner |
| 123 | Result acquirer |
| 124 | Training part |
| 125 | Communication controller |
| 126 | Storage |
| 131 | CPU |
| 132 | ROM |
| 133 | RAM |
| 134 | HDD/SSD |
| 135 | Device connection I/F |
| 136 | Communication I/F |
| 151 | First psychological state estimator |
| 152 | Second psychological state estimator |
| 153 | Psychological state level estimator |
| 155 | Reward Acquirer |
| 156 | Value updater |
| 157 | Training data storage |
| 158 | Error calculator |
| 159 | Trained model updater |
| 160 | Psychological state information acquisition apparatus |
| 161 | Registration information |
| 200 | User |
| 300 | Learning device |
| 301 | CPU |
| 302 | ROM |
| 303 | RAM |
| 304 | HDD/SSD |
| 305 | Device connection I/F |
| 306 | Communication I/F |
| A, A' | System bus |
| B | Biological information |
| C1 | First capacitance signal |
| C2 | Second capacitance signal |
| D1 | First pyroelectric signal |

| | |
|---|---|
| D2 | Second pyroelectric signal |
| F1a | Right shoulder frame |
| F2a | Right upper arm frame |
| F3a | Right elbow frame |
| F4a | Right forearm frame |
| F1b | Left shoulder frame |
| F2b | Left upper arm frame |
| F3b | Left elbow frame |
| F4b | Left forearm frame |
| F1c | Neck frame |
| F2c | Face frame |
| Im | Captured image |
| L | Error |
| LM | Trained model |
| Ms | Emission wave |
| Mr | Reflection wave |
| M1a | Right shoulder servomotor |
| M2a | Right upper arm servomotor |
| M3a | Right leg servomotor |
| M4a | Right upper arm servomotor |
| M1b | Left shoulder servomotor |
| M2b | Left upper arm servomotor |
| M3b | Left elbow servomotor |
| M4b | Left upper arm servomotor |
| M1c | Neck servomotor |
| M2c | Face servomotor |
| Q | Value |
| S | Tactile signal |
| s | Psychological state information |
| sa | First psychological state information |
| sb | Second psychological state information |
| a | Action |
| r | Reward |

**Claims**

1. A psychological state information acquisition apparatus, comprising:

   a camera configured to capture an image of a user;
   a biological information acquirer configured to acquire biological information of the user by using electromagnetic waves; and
   an information outputter configured to output psychological state information, the psychological state information being information related to a psychological state of the user obtained based on the image of the user captured by the camera and the biological information acquired by the biological information acquirer.

2. The psychological state information acquisition apparatus according to claim 1, further comprising:

   a selection information acquirer configured to acquire selection information related to at least one of a presence of motion of the user, a distance to the user, detection of a face of the user, or a relative angle with respect to the user;
   a first estimator configured to estimate a psychological state of the user from the captured image and output first psychological state information;
   a second estimator configured to estimate a psychological state of the user from the biological information and output second psychological state information; and
   a selector configured to consider the first psychological state information and the second psychological state information to select the psychological state information of the user based on the selection information.

3. The psychological state information acquisition apparatus according to claim 2, wherein

the first estimator estimates the psychological state by a trained model to which the image captured by the camera is input and which outputs the first psychological state information, and

the second estimator estimates the psychological state by a trained model to which the biological information acquired by the biological information acquirer is input and which outputs the second psychological state information.

4. The psychological state information acquisition apparatus according to claim 1, further comprising:

a selection information acquirer configured to acquire selection information related to at least one of a presence of motion of the user, a distance to the user, detection of a face of the user, or a relative angle with respect to the user; and

a third estimator configured to estimate the psychological state of the user and output third psychological state information based on a trained model to which the image captured by the camera, the biological information acquired by the biological information acquirer, and the selection information are input and which outputs the psychological state information.

5. The psychological state information acquisition apparatus according to any one of claims 1 to 4, wherein the biological information acquirer acquires the biological information by using electromagnetic waves having a frequency of 300 MHz or more and 300 GHz or less.

6. The psychological state information acquisition apparatus according to any one of claims 1 to 4, wherein the camera captures an image of the user by using light having a wavelength of 360 nm or more and 2500 nm or less.

7. The psychological state information acquisition apparatus according to any one of claims 1 to 4, further comprising a near-infrared light path filter for shielding visible light and transmitting near-infrared light, wherein the camera captures an image of the user using light transmitted through the near-infrared light path filter.

8. The psychological state information acquisition apparatus according to any one of claims 1 to 4, further comprising:

an authenticator configured to perform personal authentication of the user; and

a controller configured to control a start of at least one of the capturing of the image by the camera and the acquiring of the biological information by the biological information acquirer based on an authentication result by the authenticator.

9. A robot comprising the psychological state information acquisition apparatus according to claim 1.

# FIG.1

# FIG.2

# FIG.3

# FIG.4

# FIG.5

FIG.6

EP 4 691 366 A1

# FIG.7

EP 4 691 366 A1

# FIG.8

```
                                                    ⌐300
         LEARNING DEVICE
      ⌐301                        ⌐304
   ┌──────────┐              ┌──────────┐
   │   CPU    │              │ HDD/SSD  │
   └──────────┘              └──────────┘
      ⌐302                        ⌐305
   ┌──────────┐              ┌──────────────┐
   │   ROM    │              │   DEVICE     │
   │          │              │CONNECTION I/F│
   └──────────┘              └──────────────┘
      ⌐303                        ⌐306
   ┌──────────┐              ┌──────────────┐
   │   RAM    │              │COMMUNICATION │
   │          │              │    I/F       │
   └──────────┘              └──────────────┘
                    A'
```

# FIG.9

FIG.9

**ENVIRONMENT**

| 200 |
| USER |

| 300 |

**LEARNING DEVICE**

124

**FIRST ESTIMATOR** ~107

Im

**FIRST PSYCHOLOGICAL STATE ESTIMATOR** ~151

sa

**SECOND ESTIMATOR** ~108

B

**SECOND PSYCHOLOGICAL STATE ESTIMATOR** ~152

sb

**SELECTOR** ~109

s

**INFOR-MATION OUTPUTTER** ~110

s

**TRAINING PART**

**REWARD ACQUIRER** ~155

REWARD

r

**VALUE UPDATER** ~156

VALUE Q

**RESULT ACQUIRER** ~123

**PSYCHOLOGICAL STATE LEVEL ESTIMATOR** ~153

**STORAGE** ~126

**ROBOT** ~100

ACTION a

**COMMUNICATION CONTROLLER** ~125

**ACTION DETERMINER** ~122

EP 4 691 366 A1

# FIG.10

# FIG.11

# FIG.12

DETECT PRESENCE OF USER

ACQUIRE CAPTURED IMAGE OF USER — S11

ESTIMATE FIRST PSYCHOLOGICAL STATE OF USER BASED ON CAPTURED IMAGE OF USER — S12

ACQUIRE BIOLOGICAL INFORMATION OF USER — S13

ESTIMATE SECOND PSYCHOLOGICAL STATE OF USER BASED ON BIOLOGICAL INFORMATION OF USER — S14

ACQUIRE SELECTION INFORMATION — S15

SELECT EITHER FIRST PSYCHOLOGICAL STATE INFORMATION OR SECOND PSYCHOLOGICAL STATE INFORMATION AS PSYCHOLOGICAL STATE INFORMATION BASED ON SELECTION INFORMATION — S16

OUTPUT SELECTED PSYCHOLOGICAL STATE INFORMATION TO ACTION DETERMINER — S17

DETERMINE CERTAIN ACTION SUITABLE FOR PSYCHOLOGICAL STATE INFORMATION — S18

COMMAND TO EXECUTE ACTION THAT INDUCES FAVORABLE RESPONSE OF USER — S19

END? — S20

NO

YES

END

# FIG.13

START

S20

SELECT PSYCHOLOGICAL
STATE INFORMATION OF USER

}
ESTIMATION
PHASE

S21

DETERMINE ACTION OF ROBOT
CORRESPONDING TO PSYCHOLOGICAL
STATE INFORMATION OF USER
BASED ON VALUE OF ACTION

→ PERFORM
ACTION IN S19

S22

ACQUIRE RESPONSE EVALUATION
OF USER'S RESPONSE AS
RESULT OF ROBOT'S ACTION

S23

ACQUIRE REWARD CORRESPONDING TO
ACTION BASED ON RESPONSE
EVALUATION OF USER'S RESPONSE

}
TRAINING
PHASE

S24

UPDATE VALUE OF ROBOT'S ACTION
CORRESPONDING TO PSYCHOLOGICAL
STATE OF USER BASED ON REWARD

# FIG.14

LEARNING DEVICE 300

ENVIRONMENT

USER 200

FIRST ESTIMATOR 107
- FIRST PSYCHOLOGICAL STATE ESTIMATOR 151 → sa

SECOND ESTIMATOR 108
- SECOND PSYCHOLOGICAL STATE ESTIMATOR 152 → sb

RESULT ACQUIRER 123
- PSYCHOLOGICAL STATE LEVEL ESTIMATOR 153

SELECTOR 109 → s

INFORMATION OUTPUTTER 110 → s

TRAINING PART 124
- TRAINING DATA STORAGE 157
- ERROR CALCULATOR 158
- TRAINED MODEL UPDATER 159

ERROR L
STORAGE 126

ROBOT 100

ACTION a ← COMMUNICATION CONTROLLER 125

ACTION DETERMINER 122

Im    B

EP 4 691 366 A1

# FIG.15

<PROCESSING IN ESTIMATOR (SUPERVISED LEARNING)>

START

**S30**

CALCULATE ERROR IN VALUE OF ACTION
BASED ON TRAINING DATA OR RESPONSE
EVALUATION OF USER'S RESPONSE

**S31**

UPDATE PARAMETER OF
TRAINING MODEL BASED ON ERROR

TRAINING PHASE

**S32**

SELECT PSYCHOLOGICAL
STATE INFORMATION OF USER

**S33**

DETERMINE ACTION OF ROBOT
CORRESPONDING TO PSYCHOLOGICAL
STATE OF INFORMATION OF USER
BASED ON VALUE OF ACTION

ESTIMATION PHASE

PERFORM
ACTION IN S19

**S34**

ACQUIRE RESPONSE EVALUATION
OF USER'S RESPONSE AS
RESULT OF ROBOT'S ACTION

TRAINING PHASE

# FIG.16

# FIG.17

200

100

# FIG.18

FIG.19

# FIG.20

DETECT PRESENCE OF USER

ACQUIRE CAPTURED IMAGE OF USER — S41

ACQUIRE BIOLOGICAL INFORMATION OF USER — S42

ACQUIRE SELECTION INFORMATION — S43

ESTIMATE USER'S PSYCHOLOGICAL STATE BASED ON CAPTURED IMAGE OF USER, BIOLOGICAL INFORMATION OF USER, AND SELECTION INFORMATION — S44

OUTPUT ESTIMATED PSYCHOLOGICAL STATE INFORMATION TO ACTION DETERMINER — S45

DETERMINE CERTAIN ACTION SUITABLE FOR PSYCHOLOGICAL STATE INFORMATION — S46

COMMAND TO EXECUTE ACTION THAT INDUCES FAVORABLE RESPONSE OF USER — S47

END? — S48

NO

YES

END

## INTERNATIONAL SEARCH REPORT

| International application No. |
| --- |
| **PCT/JP2024/012526** |

| A. | CLASSIFICATION OF SUBJECT MATTER |
| --- | --- |

*A61B 5/16*(2006.01)i; *B25J 13/08*(2006.01)i
FI:    A61B5/16 100; B25J13/08

According to International Patent Classification (IPC) or to both national classification and IPC

| B. | FIELDS SEARCHED |
| --- | --- |

Minimum documentation searched (classification system followed by classification symbols)

A61B5/16; B25J13/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2022-114906 A (NTT DOCOMO, INC.) 08 August 2022 (2022-08-08) paragraphs [0011]-[0035], fig. 1-5 | 1, 5-9 |
| A | | 2-4 |
| Y | JP 2006-127057 A (CANON KABUSHIKI KAISHA) 18 May 2006 (2006-05-18) paragraph [0009], fig. 1 | 1, 5-9 |
| A | | 2-4 |
| Y | JP 2020-140609 A (KDDI CORPORATION) 03 September 2020 (2020-09-03) paragraphs [0020]-[0023] | 1, 5-9 |
| Y | JP 2020-48043 A (KABUSHIKI KAISHA TOSHIBA) 26 March 2020 (2020-03-26) paragraphs [0029]-[0032] | 6-7 |
| Y | paragraphs [0035]-[0036] | 8 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **26 April 2024** | **14 May 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
|---|
| **PCT/JP2024/012526** |

**C.     DOCUMENTS CONSIDERED TO BE RELEVANT**

| Category* | Citation of document, with indication, where appropriate, of the relevant passages | Relevant to claim No. |
|---|---|---|
| Y | JP 2017-144225 A (PANASONIC INTELLECTUAL PROPERTY MANAGEMENT CO., LTD.) 24 August 2017 (2017-08-24)<br>paragraphs [0167]-[0173] | 9 |
| A | JP 2021-37287 A (TOSHO ESTATE CO., LTD.) 11 March 2021 (2021-03-11)<br>paragraphs [0064]-[0110] | 1-9 |
| A | US 2022/0091670 A1 (HEWLETT-PACKARD DEVELOPMENT COMPANY, L.P.) 24 March 2022 (2022-03-24)<br>entire text, all drawings | 2-4 |

Form PCT/ISA/210 (second sheet) (July 2022)

**INTERNATIONAL SEARCH REPORT**
Information on patent family members

International application No.

**PCT/JP2024/012526**

| Patent document cited in search report | | | Publication date (day/month/year) | Patent family member(s) | Publication date (day/month/year) |
|---|---|---|---|---|---|
| JP | 2022-114906 | A | 08 August 2022 | (Family: none) | |
| JP | 2006-127057 | A | 18 May 2006 | WO 2006/046723 A1 p. 2, line 26 to p. 3, line 2, fig. 1 US 2007/0270664 A1 paragraphs [0009]-[0016], fig. 1 | |
| JP | 2020-140609 | A | 03 September 2020 | (Family: none) | |
| JP | 2020-48043 | A | 26 March 2020 | (Family: none) | |
| JP | 2017-144225 | A | 24 August 2017 | US 2017/0231544 A1 paragraphs [0206]-[0212] CN 107088071 A paragraphs [0276]-[0282] | |
| JP | 2021-37287 | A | 11 March 2021 | WO 2021/040025 A1 paragraphs [0064]-[0110] US 2022/0296162 A1 paragraphs [0122]-[0185] KR 10-2022-0056210 A paragraphs [0079]-[0143] CN 114466618 A paragraphs [0125]-[0191] | |
| US | 2022/0091670 | A1 | 24 March 2022 | WO 2020/251585 A1 entire text, all drawings CN 113924542 A entire text, all drawings | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020140609 A

- JP 2023054113 A